(19) Europäisches Patentamt
European Patent Office
Office européen des brevets

(11) EP 1 539 765 B1

(12) **EUROPEAN PATENT SPECIFICATION**

(45) Date of publication and mention of the grant of the patent:
**10.12.2008 Bulletin 2008/50**

(21) Application number: **03788214.9**

(22) Date of filing: **13.08.2003**

(51) Int Cl.:
***C07D 487/08*** *(2006.01)* ***A61K 31/551*** *(2006.01)*
***A61P 25/00*** *(2006.01)* ***A61P 1/04*** *(2006.01)*

(86) International application number:
**PCT/SE2003/001277**

(87) International publication number:
**WO 2004/016617 (26.02.2004 Gazette 2004/09)**

(54) **BIARYL DIAZABICYCLOALKANE AMIDES AS NICOTINIC ACETYLCHOLINE AGONISTS**

BIARYLDIAZABICYCLOALKANAMIDE ALS NICOTINISCHE ACETYLCHOLINAGONISTEN

BIARYL-DIAZABICYCLOALCANAMIDES UTILISES COMME AGONISTES NICOTINIQUES DE L'ACETYLCHOLINE

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **14.08.2002 SE 0202430**

(43) Date of publication of application:
**15.06.2005 Bulletin 2005/24**

(73) Proprietor: **AstraZeneca AB**
**151 85 Södertälje (SE)**

(72) Inventors:
- **ERNST, Glen,**
  **AstraZeneca Wilmington**
  **Wilmington, DE 19850-5437 (US)**
- **PHILLIPS, Eifion**
  **Boothwyn, PA 19061 (US)**
- **SCHMIESING, Richard,**
  **AstraZeneca Wilmington**
  **Wilmington, DE 19850-5437 (US)**

(56) References cited:
**WO-A1-00/58311**
**FR-A1- 2 809 731**
**FR-A1- 2 809 732**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 539 765 B1

## Description

### Technical Field

This invention relates to diazabicycloalkane amides or pharmaceutically-acceptable salts thereof, processes for preparing them, pharmaceutical compositions containing them and their use in therapy. The invention also relates to compounds that are ligands for nicotinic acetylcholine receptors (nAChRs).

### Background of the Invention

The use of compounds which bind nicotinic acetylcholine receptors in the treatment of a range of disorders involving reduced cholinergic function such as Alzheimer's disease, cognitive or attention disorders, anxiety, depression, smoking cessation, neuroprotection, schizophrenia, analgesia, Tourette's syndrome, and Parkinson's disease has been discussed in McDonald et al. (1995) "Nicotinic Acetylcholine Receptors: Molecular Biology, Chemistry and Pharmacology", Chapter 5 in Annual Reports in Medicinal Chemistry, vol. 30, pp. 41-50, Academic Press Inc., San Diego, CA; and in Williams et al. (1994) "Neuronal Nicotinic Acetylcholine Receptors," Drug News & Perspectives, vol. 7, pp. 205-223. WO 00/58311, FR 2809731 and FR 2809732 disclose respectively the production and therapeutic use of 1,4-diazabicyclo [3.2.2]nonane carboxylate and carboxamide derivatives, 1,4-diazabicyclo[3.2.2]nonane phenylisoxazole derivatives and 1,4-diazabicyclo[3.2.2]nonane 2-phenylthiazole derivatives.

### Disclosure of the Invention

We have invented compounds of formula I:

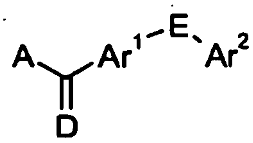

I

wherein:

A is a moiety of formula II:

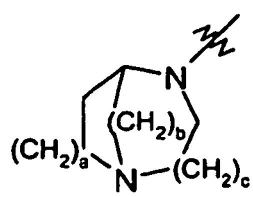

II

D is oxygen or sulfur;
E is a single bond, oxygen, sulfur, or $NR^3$;
$Ar^1$ is a 5- or 6-membered aromatic heterocyclic ring having 1, 2 or 3 heteroatoms selected from nitrogen, oxygen or sulfur where not more than one of said heteroatoms is oxygen or sulfur, or
$Ar^1$ is phenyl;
$Ar^2$ is a 5- or 6-membered aromatic heterocyclic ring having 1, 2 or 3 heteroatoms selected from nitrogen, oxygen or sulfur where not more than one of said heteroatoms is oxygen or sulfur, or
$Ar^2$ is phenyl, or
$Ar^2$ is an 8- or 9-, or 10-membered fused aromatic carbocyclic ring or fused aromatic heterocyclic ring having 1, 2 or 3 heteroatoms selected from nitrogen, oxygen or sulfur where not more than one of said heteroatoms is oxygen or sulfur, or an 8- or 9-, or 10-membered aromatic carbocyclic ring;
the rings $Ar^1$ and $Ar^2$ are substituted with 0, 1, 2 or 3 substituents selected from: halogen, $C_{1-4}$alkyl, $C_{2-4}$alkenyl, $C_{2-4}$alkynyl, CN, $NO_2$, $CF_3$ $NR^1R^2$, $CH_2NR^1R^2$, $OR^2$, $CH_2OR^2$ or $CO_2R^3$;
$R^1$ and $R^2$ at each occurrence are independently selected from hydrogen, $C_{1-4}$alkyl, aryl, heteroaryl, $C(O)R^3$, C(O)

$NHR^3$, $CO_2R^3$ or $SO_2R^3$, or
$R^1$ and $R^2$ in combination is $-(CH_2)_jG(CH_2)_k-$ wherein G is oxygen, sulfur, $NR^3$, or a bond;
a, b and c are each 1 or 2;
j is 2, 3 or 4;
k is 0, 1 or 2, and
$R^3$ at each occurrence is independently selected from hydrogen, $C_{1-4}$alkyl, aryl, or heteroaryl;
or a diastereoisomer, enantiomer or pharmaceutically-acceptable salt thereof.

One embodiment of the invention comprises compounds wherein D is oxygen.
Another embodiment of the invention comprises compounds wherein E is a single bond.
Yet another embodiment of the invention comprises compounds wherein E is oxygen or $NR^3$.
A particular embodiment of the invention comprises compounds wherein A is

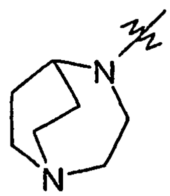

II

or a diastereoisomer, enantiomer or pharmaceutically-acceptable salt thereof.

Particular compounds of the invention are those wherein $Ar^1$ is a 5- or 6-membered aromatic heterocyclic ring having 1 or 2 heteroatoms selected from nitrogen, oxygen or sulfur where not more than one of said heteroatoms is oxygen or sulfur, or $Ar^1$ is phenyl.
Particular compounds of the invention are also those wherein $Ar^1$ is a benzene ring, furan ring or thiophene ring.
Particular compounds of the invention are also those wherein $Ar^2$ is a 5- or 6-membered aromatic heterocyclic ring having 1 or 2 heteroatoms selected from nitrogen, oxygen or sulfur where not more than one of said heteroatoms is oxygen or sulfur, or a phenyl.
Particular compounds of the invention are also those wherein $Ar^2$ is a benzene ring, furan ring, thiophene ring, or pyridine ring.
Particular compounds of the invention are also those wherein the $-EAr^2$ and the C(=D)A moieties on $Ar^1$ are positioned in a 1,3-relationship relative to each other.
Particular compounds of the invention are also those wherein $Ar^1$ or $Ar^2$ is substituted with 0 or 1 substituents selected from: halogen, $C_{1-4}$alkyl, $C_{2-4}$alkenyl, $C_{2-4}$alkynyl, CN, $NO_2$, $NR^1R^2$, $CH_2NR^1R^2$, $OR^3$, $CH_2OR^3$, $CO_2R^3$, and $CF_3$.
Particular compounds of the invention are also those wherein A is a moiety of formula II:

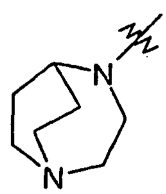

II

D is oxygen;
E is a single bond;
$Ar^1$ is a 5- or 6-membered aromatic heterocyclic ring having 1, 2 or 3 heteroatoms selected from nitrogen, oxygen or sulfur where not more than I of said heteroatoms is oxygen or sulfur, or
$Ar^1$ is phenyl
$Ar^2$ is a 5- or 6-membered aromatic heterocyclic ring having 1, 2 or 3 heteroatoms selected from nitrogen, oxygen or sulfur where not more than I of said heteroatoms is oxygen or sulfur, or
$Ar^2$ is phenyl,

or a diastereoisomer, enantiomer or pharmaceutically-acceptable salt thereof.

Still more particular compounds of the invention are those wherein $Ar^1$ is a benzene ring, furan ring or thiophene ring.

Particular compounds of the invention are also having the groups $-EAr^2$ and -C(=O)A, positioned in a 1,3-relationship relative to each other and wherein $Ar^2$ has 0 or 1 substituents selected from: halogen, $C_{1-4}$alkyl, $C_{2-4}$alkenyl, $C_{2-4}$alkynyl, CN, $NO_2$ $NR^1R^2$, $CH_2NR^1R^2$, $OR^1$, $CH_2OR^1$, $CO_2R^3$, and $CF_3$.

Most particular compounds of the invention include:

(1,4-diazabicyclo[3.2.2]non-4-yl)(biphenyl-3-yl)methanone;
(1,4-diazabicyclo[3.2.2]non-4-yl)(3-(2-pyridyl)phenyl)methanone;
(1,4-diazabicyclo[3.2.2]non-4-yl)(3-(3-pyridyl)phenyl)methanone;
(1,4-diazabicyclo[3.2.2]non-4-yl)(3-(4-pyridyl)phenyl)methanone;
(1,4-diazabicyclo[3.2.2]non-4-yl)(3-(furan-2-yl)phenyl)methanone;
(1,4-diazabicyclo[3.2.2]non-4-yl)(3-(furan-3-yl)phenyl)methanone;
(1,4-diazabicyclo[3.2.2]non-4-yl)(3-(thiophen-2-yl)phenyl)methanone;
(1,4-diazabicyclo[3.2.2]non-4-yl)(3-(thiophen-3-yl)phenyl)methanone;
(1,4-diazabicyclo[3.2.2]non-4-yl)(biphenyl-4-yl)methanone;
(1,4-diazabicyclo[3.2.2]non-4-yl)(4-(2-pyridyl)phenyl)methanone;
(1,4-diazabicyclo[3.2.2]non-4-yl)(4-(3-pyridyl)phenyl)methanone;
(1,4-diazabicyclo[3.2.2]non-4-yl)(4-(4-pyridyl)phenyl)methanone;
(1,4-diazabicyclo[3.2.2]non-4-yl)(4-(furan-2-yl)phenyl)methanone;
(1,4-diazabicyclo[3.2.2]non-4-yl)(4-(furan-3-yl)phenyl)methanone;
(1,4-diazabicyclo[3.2.2]non-4-yl)(4-(thiophen-2-yl)phenyl)methanone;
(1,4-diazabicyclo[3.2.2]non-4-yl)(4-(thiophen-3-yl)phenyl)methanone;
(1,4-diazabicyclo[3.2.2]non-4-yl)(5-phenylfuran-2-yl)methanone;
(1,4-diazabicyclo[3.2.2]non-4-yl)(5-(2-pyridyl)furan-2-yl)methanone;
(1,4-diazabicyclo[3.2.2]non-4-yl)(5-(3-pyridyl)furan-2-yl)methanone;
(1,4-diazabicyclo[3.2.2]non-4-yl)(5-(4-pyridyl)furan-2-yl)methanone;
(1,4-diazabicyclo[3.2.2]non-4-yl)(5-(furan-2-yl)furan-2-yl)methanone;
(1,4-diazabicyclo[3.2.2]non-4-yl)(5-(furan-3-yl)furan-2-yl)methanone;
(1,4-diazabicyclo[3.2.2] non-4-yl)(5-(thiophen-2-yl)furan-2-yl)methanone;
(1,4-diazabicyclo[3.2.2]non-4-yl)(5-(thiophen-3-yl)furan-2-yl)methanone;
(1,4-diazabicyclo[3.2.2]non-4-yl)(5-phenylthiophen-3-yl)methanone otherwise named (1,4-diazabicyclo[3.2.2]non-4-yl)(2-phenylthiophen-4-yl)methanone;
(1,4-diazabicyclo[3.2.2]non-4-yl)(5-(2-pyridyl)thiophen-3-yl)methanone otherwise named (1,4-diazabicyclo[3.2.2] non-4-yl)(2-(2-pyridyl)thiophen-4-yl)methanone;
(1,4-diazabicyclo[3.2.2]non-4-yl)(5-(3-pyridyl)thiophen-3-yl)methanone otherwise named (1,4-diazabicyclo[3.2.2] non-4-yl)(2-(3-pyridyl)thiophen-4-yl)methanone;
(1,4-diazabicyclo[3.2.2]non-4-yl)(5-(4-pyridyl)thiophen-3-yl)methanone otherwise named (1,4-diazabicyclo[3.2.2] non-4-yl)(2-(4-pyridyl)thiophen-4-yl)methanone;
(1,4-diazabicyclo[3.2.2]non-4-yl)(5-(furan-2-yl)thiophen-3-yl)methanone otherwise named (1,4-diazabicyclo[3.2.2] non-4-yl)(2-(furan-2-yl)thiophen-4-yl)methanone;
(1,4-diazabicyclo[3.2.2]non-4-yl)(5-(furan-3-yl)thiophen-3-yl)methanone otherwise named (1,4-diazabicyclo[3.2.2] non-4-yl)(2-(furan-3-yl)thiophen-4-yl)methanone;
(1,4-diazabicyclo[3.2.2]non-4-yl)(5-(thiophen-2-yl)thiophen-3-yl)methanone otherwise named (1,4-diazabicyclo [3.2.2]non-4-yl)(2-(thiophen-2-yl)thiophen-4-yl)methanone;
(1,4-diazabicyclo[3.2.2]non-4-yl)(5-(thiophen-3-yl)thiophen-3-yl)methanone otherwise named (1,4-diazabicyclo [3.2.2]non-4-yl)(2-(thiophen-3-yl)thiophen-4-yl)methanone;
(1,4-diazabicyclo[3.2.2]non-4-yl)(4-phenylfuran-2-yl)methanone;
(1,4-diazabicyclo[3.2.2]non-4-yl)(4-(2-pyridyl)furan-2-yl)methanone;
(1,4-diazabicyclo[3.2.2]non-4-yl)(4-(3-pyridyl)furan-2-yl)methanone;
(1,4-diazabicyclo[3.2.2]non-4-yl)(4-(4-pyridyl)furan-2-yl)methanone;
(1,4-diazabicyclo[3.2.2]non-4-yl)(4-(furan-2-yl)furan-2-yl)methanone;
(1,4-diazabicyclo[3.2.2]non-4-yl)(4-(furan-3-yl)furan-2-yl)methanone;
(1,4-diazabicyclo[3.2.2]non-4-yl)(4-(thiophen-2-yl)furan-2-yl)methanone, and
(1,4-diazabicyclo[3.2.2]non-4-yl)(4-(thiophen-3-yl)furan-2-yl)methanone.

Other compounds of the invention are:

(1,4-diazabicyclo[3.2.2]non-4-yl)(5-phenylthiophen-2-yl)methanone;
(1,4-diazabicyclo[3.2.2]non-4-yl)(5-(2-pyridyl)thiophen-2-yl)methanone;
(1,4-diazabicyclo[3.2.2]non-4-yl)(5-(3-pyridyl)thiophen-2-yl)methanone;
(1,4-diazabicyclo[3.2.2]non-4-yl)(5-(4-pyridyl)thiophen-2-yl)methanone;
(1,4-diazabicyclo[3.2.2]non-4-yl)(5-(furan-2-yl)thiophen-2-yl)methanone;
(1,4-diazabicyclo[3.2.2]non-4-yl)(5-(furan-3-yl)thiophen-2-yl)methanone;
(1,4-diazabicyclo[3.2.2]non-4-yl)(5-(thiophen-2-yl)thiophen-2-yl)methanone, and
(1,4-diazabicyclo[3.2.2]non-4-yl)(5-(thiophen-3-yl)thiophen-2-yl)methanone.

Yet other compounds of the invention are:

(1,4-diazabicyclo[3.2.2]non-4-yl)(2-phenylfuran-4-yl)methanone ;
(1,4-diazabicyclo[3.2.2]non-4-yl)(2-(2-pyridyl)furan-4-yl)methanone;
(1,4-diazabicyclo[3.2.2]non-4-yl)(2-(3-pyridyl)furan-4-yl)methanone;
(1,4-diazabicyclo[3.2.2]non-4-yl)(2-(4-pyridyl)furan-4-yl)methanone;
(1,4-diazabicyclo[3.2.2]non-4-yl)(2-(furan-2-yl)furan-4-yl)methanone;
(1,4-diazabicyclo[3.2.2]non-4-yl)(2-(furan-3-yl)furan-4-yl)methanone;
(1,4-diazabicyclo[3.2.2]non-4-yl)(2-(thiophen-2-yl)furan-4-yl)methanone;
(1,4-diazabicyclo[3.2.2]non-4-yl)(2-(thiophen-3-yl)furan-4-yl)methanone;
(1,4-diazabicyclo[3.2.2]non-4-yl)(4-phenylthiophen-2-yl)methanone;
(1,4-diazabicyclo[3.2.2]non-4-yl)(4-(2-pyridyl)thiophen-2-yl)methanone;
(1,4-diazabicyclo[3.2.2]non-4-yl)(4-(3-pyridyl)thiophen-2-yl)methanone;
(1,4-diazabicyclo[3.2.2]non-4-yl)(4-(4-pyridyl)thiophen-2-yl)methanone;
(1,4-diazabicyclo[3.2.2]non-4-yl)(4-(furan-2-yl)thiophen-2-yl)methanone;
(1,4-diazabicyclo[3.2.2]non-4-yl)(4-(furan-3-yl)thiophen-2-yl)methanone;
(1,4-diazabicyclo[3.2.2]non-4-yl)(4-(thiophen-2-yl)thiophen-2-yl)methanone, and
(1,4-diazabicyclo[3.2.2]non-4-yl)(4-(thiophen-3-yl)thiophen-2-yl)methanone.

All embodiments and particular forms of the invention encompass all enantiomers, diastereoisomers and pharmaceutically-acceptable derivatives and salts of compounds thereof.

Compounds of the invention are potent ligands for nicotinic acetylcholine receptors.

Pharmaceutically-acceptable derivatives include solvates and salts. For example, the compounds of formula I can form acid addition salts with acids, such as the conventional pharmaceutically-acceptable acids, for example, maleic, hydrochloric, hydrobromic, phosphoric, acetic, fumaric, salicylic, citric, lactic, mandelic, tartaric and methanesulfonic acids.

Compounds of the invention are useful in the treatment or prophylaxis of human diseases or conditions in which activation of the $\alpha$7 nicotinic receptor is beneficial as well as in the treatment or prophylaxis of psychotic disorders or intellectual impairment disorders. Examples of such conditions, diseases or disorders are Alzheimers disease, learning deficit, cognition deficit, attention deficit, memory loss, Attention Deficit Hyperactivity Disorder, Anxiety, schizophrenia, mania or manic depression, Parkinson's disease, Huntington's disease, Tourette's syndrome, neurodegenerative disorders in which there is loss of cholinergic synapse, jetlag, cessation of smoking, nicotinic addiction including that resulting from exposure to products containing nicotine, pain, for ulcerative colitis and irritable bowel disease.

As used herein, unless otherwise indicated, "$C_{1-4}$alkyl" includes but is not limited to methyl, ethyl, n-propyl, n-butyl, i-propyl, i-butyl, t-butyl, s-butyl moieties, whether alone or part of another group, $C_{1-4}$alkyl groups may be straight-chained or branched, and $C_{3-4}$ alkyl groups include the cyclic alkyl moieties cyclopropyl and cyclobutyl. Alkyl groups referred to herein may have 1, 2 or 3 halogen substituents.

As used herein, unless otherwise indicated, "$C_{2-4}$alkenyl" includes but is not limited to 1-propenyl, 2-propenyl, 1-butenyl, 2-butenyl and 3-butenyl.

As used herein, unless otherwise indicated, "$C_{2-4}$alkynyl" includes but is not limited to ethynyl, 1-propynyl, 2-propynyl, 1-butynyl, 2-butynyl and 3-butynyl.

As used herein, unless otherwise indicated, aryl refers to a phenyl ring which may have 1, 2 or 3 substituents selected from: halogen, $C_{1-4}$alkyl, $C_{2-4}$alkenyl, $C_{2-4}$alkynyl, $C_{1-4}$alkyl, CN, $NO_2$, and $CF_3$.

As used herein, unless otherwise indicated, heteroaryl refers to a 5- or 6-membered aromatic or heteroaromatic ring having 1, 2 or 3 heteroatoms selected from nitrogen oxygen and sulfur, provided that heteroaromatic rings contains at least one nitrogen, oxygen, or sulfur atom.

As used herein, unless otherwise indicated, halogen refers to fluorine, chlorine, bromine, or iodine.

**Methods of Preparation**

In the reaction schemes and text that follow, A, E, $Ar^1$, and $Ar^2$ unless otherwise indicated, are as defined above for formula I.

The compounds of formula I in which E represent a single bond may be prepared according to the methods outlined in Scheme 1.

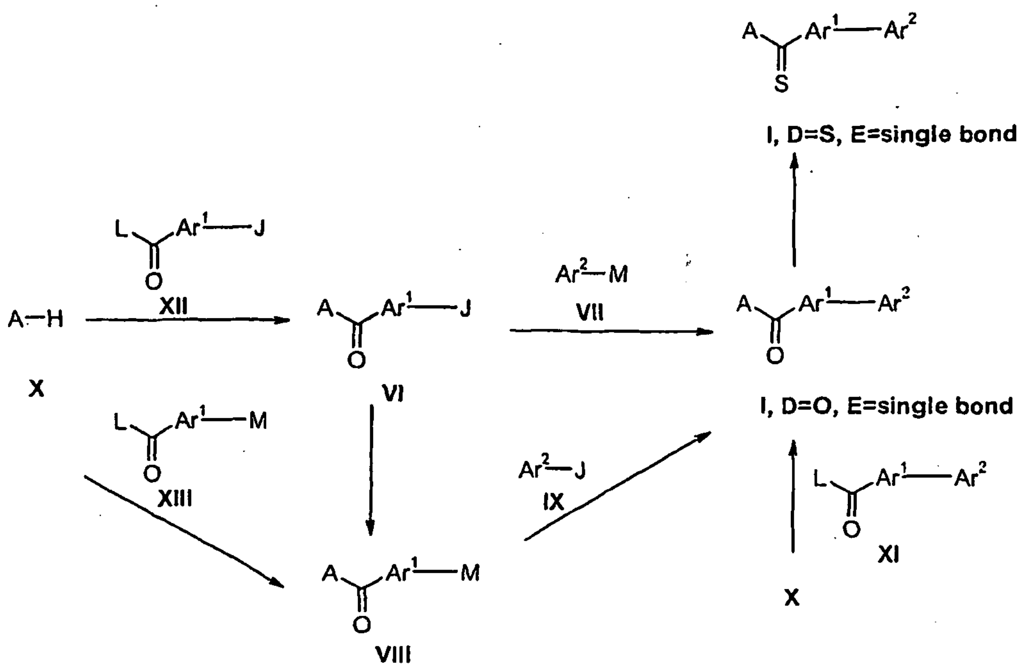


**Scheme 1**

Compounds of formula I wherein D is oxygen and E is a single bond may be prepared from compounds of formula VI wherein J is a halogen or an $OSO_2CF_3$ substituent at the position of ring $Ar^1$ at which the bond to ring $Ar^2$ is formed, by reaction with an appropriate organometallic compound of formula VII in the presence of a suitable organometallic catalyst and solvent. Suitable compounds of formula VII include boronic acids, in which M is $B(OH)_2$ and organotin compounds, in which M is a suitable trialkylstannyl group, for example trimethylstannyl or tri-n-butylstannyl. Suitable organometallic catalysts include palladium (0) complexes, for example tetrakis(triphenylphosphine)palladium(0) or a combination of tris(dibenzylideneacetone)dipalladium(0) and a suitable triarylphosphine or triarylarsine ligand, for example triphenylphosphine, tri(o-tolyl)phosphine or triphenylarsine.

Suitable solvents include inert ether solvents, for example 1,2-dimethoxyethane, tetrahydrofuran, or 1,4-dioxane, or alcohols, such as ethanol, or mixtures thereof. If the compound of formula VII is a boronic acid, the presence of a suitable base in addition to the other reagents is preferred. Suitable bases include sodium carbonate, cesium carbonate, and barium hydroxide. The reaction is carried out at a temperature of 0-120 ˚C, and preferably at a temperature of 60-120 ˚C.

Compounds of formula I wherein D is oxygen and E is a single bond may also be prepared from organometallic compounds of formula VIII by reaction with a compound of formula IX in which J is a halogen or $OSO_2CF_3$ in the presence of a suitable organometallic catalyst and solvent. Suitable compounds of formula VIII include boronic acids, in which M is $B(OH)_2$ and organotin compounds, in which M is a suitable trialkylstannyl group, for example trimethylstannyl or tri-n-butylstannyl.

Suitable organometallic catalysts include palladium (0) complexes, for example tetrakis(triphenylphosphine) palladium (0) or a combination of tris(dibenzylideneacetone)dipalladium (0) and a suitable triarylphosphine or triarylarsine ligand, for example triphenylphosphine, tri(o-tolyl)phosphine or triphenylarsine.

Suitable solvents include inert ether solvents, for example 1,2-dimethoxyethane, tetrahydrofuran, or 1,4-dioxane, or alcohols, such as ethanol, or mixtures thereof.

If the compound of formula VIII is a boronic acid, the presence of a suitable base in addition to the other reagents is preferred. Suitable bases include sodium carbonate, cesium carbonate, and barium hydroxide. The reaction is carried

out at a temperature of 0-120 ˚C, and preferably at a temperature of 60-120 ˚C.

Compounds of formula I wherein D is oxygen and E is a single bond may also be prepared from compounds of formula X by reaction with a suitable compound of formula XI, wherein L is a suitable leaving group, using a suitable acylation procedure. Suitable leaving groups L include: OH, halogen, Oalkyl, Oaryl, OCOalkyl, OCOaryl. A suitable acylation procedure involves treatment of a compound of formula X with a compound of formula XI at 0-120 ˚C in a suitable solvent. The presence of a base, or, when Y=OH, a coupling agent, may also be necessary for the reaction to occur. Suitable bases for the reaction include: 4-(*N,N*-dimethylamino)pyridine, pyridine, triethylamine, *N,N*-diisopropylethylamine. The preferred base is *N,N*-diisopropylethylamine. Suitable coupling agents when L=OH include: carbodiimides, for example 1,3-dicyclohexylcarbodiimide or 1-(3-dimethylaminopropyl-3-ethylcarbodiimide hydrochloride; phosphonium reagents, for example benzotriazol-1-yloxytris(dimethylamino)phosphonium hexafluorophosphate or benzotriazol-1-yloxytripyrrolidinophosphonium hexafluorophosphate; and uronium reagents, for example *O*-benzotriazol-1-yl-*N,N,N',N'*-tetramethyluronium tetrafluoroborate. The preferred coupling agent is O-benzotriazol-1-yl-*N,N,N',N'*-tetramethyluronium tetrafluoroborate. Suitable solvents for the reaction include *N,N*-dimethylformamide, dimethylsulfoxide, tetrahydrofuran, or chloroform. The preferred solvent is *N,N*-dimethylformamide. The reaction is preferably performed at a temperature of 0-50 ˚C, and most preferably at a temperature of 20-30 ˚C.

Compounds of formula I in which D is sulfur and E is a single bond may be prepared from compounds of formula I in which D is oxygen and E is a single bond by reaction with a suitable sulfide in a suitable solvent. The preferred sulfides are phosphorus sulfides, in particular 4-methoxyphenylthionophosphine sulfide dimer ("Lawesson's Reagent"), and diphosphorus pentasulfide. Suitable solvents for the reaction include aryl hydrocarbon solvents, for example toluene or xylene. The reaction is performed at a temperature of 0-200 ˚C, and preferably at a temperature of 50-180 ˚C.

Certain compounds of formula VI wherein J is halogen may be prepared from compounds of formula VI wherein J is hydrogen by reaction with a suitable halogenating agent in a suitable solvent. Suitable halogenating agents include bromine. Suitable solvents include acetic acid. The reaction is preferably performed at a temperature of 0-50 ˚C, and most preferably at a temperature of 0-25 ˚C.

Compounds of formula VI wherein J is $OSO_2CF_3$ may be prepared from compounds of formula VI wherein J is OH by reaction with trifluoromethanesulfonic anhydride or other trifluoromethanesulfonylating agent in the presence of a base and a suitable solvent. Suitable bases include pyridine, and 2,6-di-t-butylpyridine. The reaction is preferably performed at a temperature of -78 to 120 ˚C, and most preferably at a temperature of -78 to 0 ˚C.

Compounds of formula VI wherein J is hydrogen, halogen, OH, or $OSO_2CF_3$ may be prepared from compounds of formula X by reaction with a suitable compound of formula XII, wherein L is a suitable leaving group and J is hydrogen, halogen, OH, or $OSO_2CF_3$, using a suitable acylation procedure. Suitable leaving groups L include: OH, halogen, Oalkyl, Oaryl, OCOalkyl, OCOaryl. A suitable acylation procedure involves treatment of a compound of formula X with a compound of formula XII at 0-120 ˚C in a suitable solvent. The presence of a base, or, when L=OH, a coupling agent, may also be necessary for the reaction to occur. Suitable bases for the reaction include: 4-(*N,N*-dimethylamino)pyridine, pyridine, triethylamine, *N,N*-diisopropylethylamine. The preferred base is *N,N*-diisopropylethylamine. Suitable coupling agents when Y=OH include: carbodiimides, for example 1,3-dicyclohexylcarbodiimide or 1-(3-dimethylaminopropyl-3-ethylcarbodiimide hydrochloride; phosphonium reagents, for example benzotriazol-1-yloxytris(dimethylamino)phosphonium hexafluorophosphate or benzotriazol-1-yloxytripyrrolidinophosphonium hexafluorophosphate; and uronium reagents, for example *O*-benzotriazol-1-yl-*N,N,N'N'*-tetramethyluronium tetrafluoroborate. The preferred coupling agent is *O*-benzotriazol-1-yl-*N,N,N',N'*-tetramethyluronium tetrafluoroborate. Suitable solvents for the reaction include *N, N*-dimethylformamide, dimethylsulfoxide, tetrahydrofuran, or chloroform. The preferred solvent is *N,N*-dimethylformamide. The reaction is preferably performed at a temperature of 0-50 ˚C, and most preferably at a temperature of 20-30 ˚C.

Compounds of formula VIII in which M is $B(OH)_2$ may be prepared from compounds of formula VI in which J is hydrogen, halogen, or $OSO_2CF_3$ by methods known to one skilled in the art. For example compounds of formula VI in which J is hydrogen or halogen may be converted to compounds of formula VIII in which M is $B(OH)_2$ via conversion to the corresponding aryllithium or arylmagnesium compounds followed by reaction with trimethylborate and subsequent hydrolysis, of the resulting borate ester. The reaction is performed in a suitable inert solvent, for example, tetrahydrofuran. Alternatively, compounds of formula VI wherein J is halogen or $OSO_2CF_3$ may be converted to compounds of formula VIII in which M is $B(OH)_2$ via reaction with bis(pinacolato)diboron and an organometallic catalyst, followed by hydrolysis of the resulting borate ester. For typical procedures for effecting such conversions, see, for example, Organic Syntheses, 1963, Coll. Vol. 4, 68; J. Org. Chem. 1995, 60, 7508.

Compounds of formula VIII in which M is a trialkylstannyl group may be prepared from compounds of formula VI in which J is hydrogen, halogen, or $OSO_2CF_3$ by methods known to one skilled in the art. For example compounds of formula VI in which J is hydrogen or halogen may be converted to compounds of formula VIII in which M is a trialkylstannyl group via conversion to the corresponding aryllithium or arylmagnesium compounds followed by reaction with an appropriate trialkylstannyl halide. The reaction is performed in a suitable inert solvent, for example, tetrahydrofuran. The reaction is performed at a temperature of -78 ˚C to 20 ˚C, preferably at -78˚C to 0˚C. Alternatively, compounds of formula VI wherein J is halogen or $OSO_2CF_3$ may be converted to compounds of formula VIII in which M is a trialkylstannyl

group via reaction with the appropriate bis(trialkyltin). The reaction is performed in a suitable inert solvent, for example tetrahydrofuran, in the presence of a suitable organometallic catalyst, for example tetrakis(triphenylphosphine). The reaction is performed at a temperature of 0˚C to 150˚C, preferably at 20 ˚C to 100˚C.

Compounds of formula VIII wherein M is $B(OH)_2$ or a trialkylstannyl group may be prepared from compounds of formula X by reaction with a suitable compound of formula XIII, wherein L is a suitable leaving group M is $B(OH)_2$ or a trialkylstannyl group, using a suitable acylation procedure. Suitable leaving groups L include: OH, halogen, Oalkyl, Oaryl, OCOalkyl, OCOaryl. A suitable acylation procedure involves treatment of a compound of formula X with a compound of formula XIII at 0-120 ˚C in a suitable solvent. The presence of a base, or, when L=OH, a coupling agent, may also be necessary for the reaction to occur. Suitable bases for the reaction include: 4-(*N,N*-dimethylamino)pyridine, pyridine, triethylamine, *N,N*-diisopropylethylamine. The preferred base is *N,N*-diisopropylethylamine. Suitable coupling agents when L=OH include: carbodiimides, for example 1,3-dicyclohexylcarbodiimide or 1-(3-dimethylaminopropyl-3-ethylcarbodiimide hydrochloride; phosphonium reagents, for example benzotriazol-1-yloxytris(dimethylamino)phosphonium hexafluorophosphate or benzotriazol-1-yloxytripyrrolidinophosphonium hexafluorophosphate; and uronium reagents, for example *O*-benzotriazol-1-yl-*N,N,N',N'*-tetramethyluronium tetrafluoroborate. The preferred coupling agent is O-benzotriazol-1-yl-*N,N,N',N'*-tetramethyluronium tetrafluoroborate. Suitable solvents for the reaction include *N, N*-dimethylformamide, dimethylsulfoxide, tetrahydrofuran, or chloroform. The preferred solvent is *N,N*-dimethylformamide. The reaction is preferably performed at a temperature of 0-50 ˚C, and most preferably at a temperature of 20-30 ˚C.

Compounds of formula XI may be prepared from compounds of formula XII wherein J is a halogen or $OSO_2CF_3$ substituent at the position of ring $Ar^1$ at which the bond to ring $Ar^2$ is formed, by reaction with an appropriate organometallic compound of formula VII in the presence of a suitable organometallic catalyst and solvent. Suitable compounds of formula VII include boronic acids, in which M is $B(OH)_2$ and organotin compounds, in which M is a suitable trialkylstannyl group, for example trimethylstannyl or tri-n-butylstannyl. Suitable organometallic catalysts include palladium (0) complexes, for example tetrakis(triphenylphosphine)palladium (0) or a combination of tris(dibenzylidieneacetone)dipalladium (0) and a suitable triarylphosphine or triarylarsine ligand, for example triphenylphosphine, tri(o-tolyl)phosphine or triphenylarsine. Suitable solvents include inert ether solvents, for example 1,2-dimethoxyethane, tetrahydrofuran, or 1,4-dioxane, or alcohols, such as ethanol, or mixtures thereof. If the compound of formula VII is a boronic acid, the presence of a suitable base in addition to the other reagents is preferred. Suitable bases include sodium carbonate, cesium carbonate, and barium hydroxide. The reaction is carried out at a temperature of 0-120 ˚C, and preferably at a temperature of 60-120˚C.

Compounds of formula XI may also be prepared from organometallic compounds of formula XIII by reaction with a compound of formula LX in which J is a halogen or $OSO_2CF_3$ in the presence of a suitable organometallic catalyst and solvent. Suitable compounds of formula XIII include boronic acids, in which M is $B(OH)_2$ and organotin compounds, in which M is a suitable trialkylstannyl group, for example trimethylstannyl or tri-n-butylstannyl. Suitable organometallic catalysts include palladium (0) complexes, for example tetrakis(triphenylphosphine)palladium (0) or a combination of tris(dibenzylideneacetone)dipalladium (0) and a suitable triarylphosphine or triarylarsine ligand, for example triphenylphosphine, tri(o-tolyl)phosphine or triphenylarsine. Suitable solvents include inert ether solvents, for example 1,2-dimethoxyethane, tetrahydrofuran, or 1,4-dioxane, or alcohols, such as ethanol, or mixtures thereof. If the compound of formula VIII is a boronic acid, the presence of a suitable base in addition to the other reagents is preferred. Suitable bases include sodium carbonate, cesium carbonate, and barium hydroxide. The reaction is carried out at a temperature of 0-120 ˚C, and preferably at a temperature of 60-120 ˚C.

Compounds of formula VII and compounds of formula XIII are either commercially available, or may be prepared by methods known to one skilled in the art. In particular, methods are known to one skilled in the art for the conversion of aryl halides or heteroaryl halides to aryl or heteroaryl boronic acids or aryl or heteroaryl trialkylstannanes, providing methods for the conversion of compounds of formula IX in which J is halogen to compounds of formula VII and compounds of formula XII in which J is halogen to compounds of formula XIII. For example, boronic acids may be synthesized from aryl or heteroaryl halides via conversion to the aryllithium or arylmagnesium compounds followed by reaction with trimethylborate, or *via* reaction with bis(pinacolato)diboron and an organometallic catalyst, followed by hydrolysis of the resulting borate ester (see, for example, Organic Syntheses, 1963, Coll. Vol. 4, 68; J. Org. Chem. 1995, 60, 7508). Trialkylstannanes may be synthesized from aryl or heteroaryl halides via conversion to the aryllithium or arylmagnesium compounds followed by reaction with the appropriate chlorotrialkyltin, or *via* reaction with the appropriate bis(trialkyltin) and an organometallic catalyst.

The compounds of formula I in which E is oxygen, sulfur, or $NR^3$ may be prepared according to the methods outlined in Scheme 2.

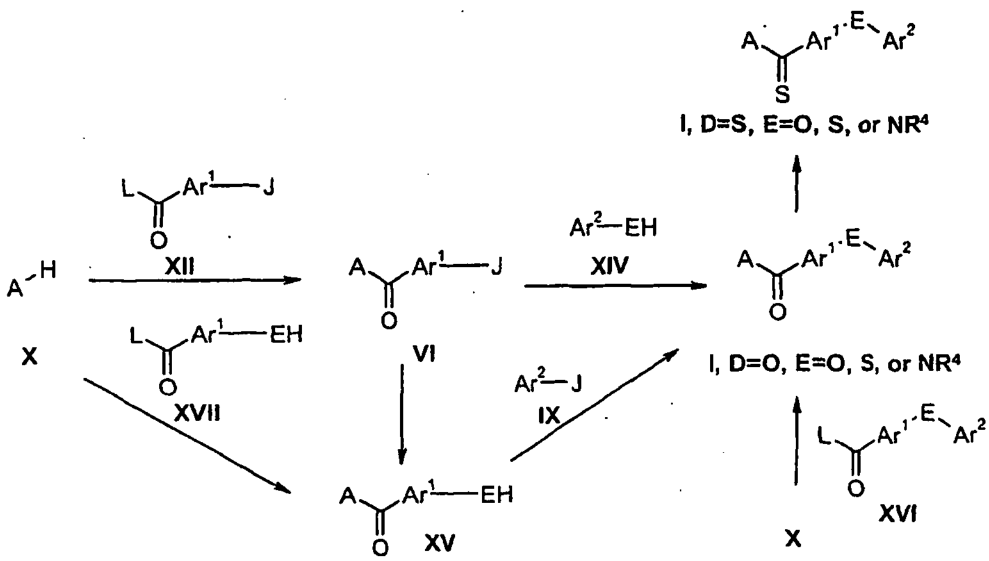

**Scheme 2**

Compounds of formula I wherein D is oxygen and E is $NR^3$ may be prepared from compounds of formula VI wherein J is a halogen or $OSO_2CF_3$ substituent at the position of ring $Ar^1$ at which the bond to nitrogen is formed, by reaction with an appropriate amine of formula XIV in which EH is $NHR^3$. The reaction may be performed by heating in an inert solvent in the presence of a suitable strong base. Suitable inert solvents include ether solvents, for example tetrahydrofuran, 1,4-dioxane, 1,2-dimethoxyethane, or di(2-methoxyethyl)ether, a hydrocarbon solvent, for example benzene or toluene, or an amide solvent, for example dimethylformamide, or *N*-methyl-2-pyrrolidinone. The preferred solvent is tetrahydrofuran. Suitable strong bases include alkali metal alkoxide or amide bases, for example sodium t-butoxide or potassium t-butoxide, lithium bis(trimethylsilyl)amide, or lithium diisopropylamide. The preferred strong base is sodium t-butoxide. The reaction may require, and is preferably performed in, the presence of an organometallic catalyst. Suitable organometallic catalysts include complexes of palladium (0) with a suitable phosphine ligand, preferably a triarylphosphine ligand, and most preferably a bidentate triarylphosphine ligand. Preferred ligands include 2,2'-bis (diphenylphosphosphino)-1,1'-binaphthyl or 1, l'-bis(diphenylphosphino)ferrocene. The catalyst may be synthesized by the combination of a suitable source of palladium (0), for example tris(dibenzylideneacetone)dipalladium (0), with the phosphine ligand, and may either be pre-formed or formed *in situ* by including the palladium source and phophine ligand in the reaction mixture. The reaction is carried out at a temperature of 0-150 °C, and preferably at a temperature of 60-120 °C.

Compounds of formula I wherein D is oxygen and E is $R^3$ may also be prepared from compounds of formula IX wherein J is a halogen or $OSO_2CF_3$ substituent at the position of ring $Ar^2$ at which the bond to nitrogen is formed, by reaction with an appropriate amine of formula XV in which EH is $NHR^3$. The reaction may be performed by heating in an inert solvent in the presence of a suitable strong base. Suitable inert solvents include ether solvents, for example tetrahydrofuran, 1,4-dioxane, 1,2-dimethoxyethane, or di(2-methoxyethyl)ether, a hydrocarbon solvent, for example benzene or toluene, or an amide solvent, for example dimethylformamide, or *N*-methyl-2-pyrrolidinone. The preferred solvent is tetrahydrofuran. Suitable strong bases include alkali metal alkoxide or amide bases, for example sodium *t*-butoxide or potassium t-butoxide, lithium bis(trimethylsilyl)amide, or lithium diisopropylamide. The preferred strong base is sodium t-butoxide. The reaction may require, and is preferably performed in, the presence of an organometallic catalyst. Suitable organometallic catalysts include complexes of palladium (0) with a suitable phosphine ligand, preferably a triarylphosphine ligand, and most preferably a bidentate triarylphosphine ligand. Preferred ligands include 2,2'-bis (diphenylphosphino)-1,1'-binaphthyl or 1,1'-bis(diphenylphosphino)ferrocene. The catalyst may be synthesized by the combination of a suitable source of palladium (0), for example tris(dibenzylideneacetone)dipalladium (0), with the phosphine ligand, and may either be pre-formed or formed *in situ* by including the palladium source and phophine ligand in the reaction mixture. The reaction is carried out at a temperature of 0-150 °C, and preferably at a temperature of 60-120 °C.

Compounds of formula I wherein D is oxygen and E is oxygen or sulfur may be prepared from compounds of formula VI wherein J is a halogen or $OSO_2CF_3$ substituent at the position of ring $Ar^1$ at which the bond to oxygen is formed, by reaction with an appropriate compound of formula XIV in which EH is OH or SH. The reaction may be performed by heating in an inert solvent in the presence of a suitable base. The reaction may require, and is preferably

performed in, the presence of a catalyst. Suitable inert solvents include ether solvents, for example tetrahydrofuran, 1,4-dioxane, 1,2-dimethoxyethane, or di(2-methoxyethyl)ether, an amide solvent, for example dimethylformamide, or *N*-methyl-2-pyrrolidinone, or a basic heterocyclic aromatic solvent, for example pyridine. The preferred solvent is pyridine. Suitable bases include alkali metal alkoxides, or alkali metal carbonates, for example potassium carbonate. Suitable organometallic catalysts include copper or its salts, preferably copper (I). salts, and most preferably copper (I) iodide. The reaction is carried out at a temperature of 0-150 ˚C, and preferably at a temperature of 100-150 ˚C.

Compounds of formula I wherein D is oxygen and E is oxygen or sulfur may also be prepared from compounds of formula IX wherein J is a halogen or $OSO_2CF_3$ substituent at the position of ring $Ar^2$ at which the bond to nitrogen is formed, by reaction with an appropriate compound of formula XV in which EH is OH or SH. The reaction may be performed by heating in an inert solvent in the presence of a suitable base. The reaction may require, and is preferably performed in, the presence of a catalyst. Suitable inert solvents include ether solvents, for example tetrahydrofuran, 1,4-dioxane, 1,2-dimethoxyethane, or di(2-methoxyethyl)ether, an amide solvent, for example *N,N*-dimethylformamide, or *N*-methylpyrrolidinone, or a basic heterocyclic aromatic solvent, for example pyridine. The preferred solvent is pyridine. Suitable bases include alkali metal alkoxides, or alkali metal carbonates, for example potassium carbonate. Suitable organometallic catalysts include copper or its salts, preferably copper (I) salts, and most preferably copper (I) iodide. The reaction is carried out at a temperature of 0-150 ˚C, and preferably at a temperature of 100-150 ˚C.

Compounds of formula I wherein D is oxygen and E is oxygen, sulfur, or $NR^3$ may also be prepared from compounds of formula X by reaction with a suitable compound of formula XVI, wherein E is oxygen, sulfur, or $NR^3$ and L is a suitable leaving group, using a suitable acylation procedure. Suitable leaving groups L include: OH, halogen, Oalkyl, Oaryl, OCOalkyl, OCOaryl. A suitable acylation procedure involves treatment of a compound of formula X with a compound of formula XI at 0-120 ˚C in a suitable solvent. The presence of a base, or, when Y=OH, a coupling agent, may also be necessary for the reaction to occur. Suitable bases for the reaction include: 4-(*N,N*-dimethylamino)pyridine, pyridine, triethylamine, *N,N*-diisopropylethylamine. The preferred base is *N,N*-diisopropylethylamine. Suitable coupling agents when L=OH include: carbodiimides, for example 1,3-dicyclohexylcarbodiimide or 1-(3-dimethylaminopropyl-3-ethylcarbodiimide hydrochloride; phosphonium reagents, for example benzotriazol-1-yloxytris(dimethylamino)phosphonium hexafluorophosphate or benzotriazol-1-yloxytripyrrolidinophosphonium hexafluorophosphate; and uronium reagents, for example *O*-benzotriazol-1-yl-*N,N,N',N'*-tetramethyluronium tetrafluoroborate. The preferred coupling agent is O-benzotriazol-1-yl-*N,N,N',N'*-tetramethyluronium tetrafluoroborate. Suitable solvents for the reaction include *N,N*-dimethylformamide, dimethylsulfoxide, tetrahydrofuran, or chloroform. The preferred solvent is *N,N*-dimethylformamide. The reaction is preferably performed at a temperature of 0-50 ˚C, and most preferably at a temperature of 20-30 ˚C.

Compounds of formula XV wherein EH is OH, SH, or $NHR^3$ may be prepared from compounds of formula X by reaction with a suitable compound of formula XVII, wherein L is a suitable leaving group and EH is OH, SH or $NHR^3$, using a suitable acylation procedure. Suitable leaving groups L include: OH, halogen, Oalkyl, Oaryl, OCOalkyl, OCOaryl. A suitable acylation procedure involves treatment of a compound of formula X with a compound of formula XVII at 0-120 ˚C in a suitable solvent. The presence of a base, or, when L=OH, a coupling agent, may also be necessary for the reaction to occur. Suitable bases for the reaction include: 4-(*N,N*-dimethylamino)pyridine, pyridine, triethylamine, *N,N*-diisopropylethylamine. The preferred base is *N,N*-diisopropylethylamine. Suitable coupling agents when L=OH include: carbodiimides, for example 1,3-dicyclohexylcarbodiimide or 1-(3-dimethylaminopropyl-3-ethylcarbodiimide hydrochloride; phosphonium reagents, for example benzotriazol-1-yloxytris(dimethylamino)phosphonium hexafluorophosphate or benzotriazol-1-yloxytripyrrolidinophosphonium hexafluorophosphate; and uronium reagents, for example *O*-benzotriazol-1-yl-*N,N,N',N'*-tetramethyluronium tetrafluoroborate. The preferred coupling agent is *O*-benzotriazol-1-yl-*N,N,N',N'*-tetramethyluronium tetrafluoroborate. Suitable solvents for the reaction include *N,N*-dimethylformamide, dimethylsulfoxide, tetrahydrofuran, or chloroform. The preferred solvent is *N,N*-dimethylformamide. The reaction is preferably performed at a temperature of 0-50 ˚C, and most preferably at a temperature of 20-30 ˚C.

Compounds of formula I, XIV, XV or XVII in which E is $NR^3$ and $R^3$ is an alkyl group may be prepared from compounds of the corresponding formula wherein $R^3$ is hydrogen by a suitable alkylation procedure. Typical alkylation procedures include treatment with an appropriate alkyl halide or sulfonate ester and base, for example sodium hydride, in a suitable solvent, for example *N,N*-dimethylformamide, or reductive alkylation using the appropriate aldehyde or ketone together with a suitable reducing agent in the presence of an acidic catalyst and in an inert solvent. The preferred method is reductive alkylation. Suitable reducing agents include sodium borohydride and sodium cyanoborohydride. The preferred reducing agent is sodium borohydride. Suitable inert solvents include water, methanol or ethanol. The preferred solvent is methanol. Suitable acidic catalysts include acetic acid or zinc chloride. The preferred acidic catalyst is acetic acid. The reaction is usually conducted at a temperature of 0-100 ˚C, and preferably at 20-65 ˚C.

Compounds of formula I, XIV, XV or XVII in which E is $NR^3$ and $R^3$ is an aryl or heteroaryl group may be prepared from compounds of the corresponding formula wherein $R^3$ is hydrogen by reaction with an appropriate aromatic or heteroaromatic halide or trifluoromethanesulfonate. The reaction may be performed by heating in an inert solvent in the presence of a suitable strong base. Suitable inert solvents include ether solvents, for example tetrahydrofuran, 1,4-dioxane, 1,2-dimethoxyethane, or di(2-methoxyethyl)ether, a hydrocarbon solvent, for example benzene or toluene, or

an amide solvent, for example *N,N*-dimethylformamide, or *N*-methyl-2-pyrrolidinone. The preferred solvent is tetrahydrofuran. Suitable strong bases include alkali metal alkoxide or amide bases, for example sodium t-butoxide or potassium t-butoxide, lithium bis(trimethylsilyl)amide, or lithium diisopropylamide. The preferred strong base is sodium t-butoxide. The reaction may require, and is preferably performed in, the presence of an organometallic catalyst. Suitable organometallic catalysts include complexes of palladium (0) with a suitable phosphine ligand, preferably a triarylphosphine ligand, and most preferably a bidentate triarylphosphine ligand. Preferred ligands include 2,2'-bis(diphenylphosphino)-1,1'-binaphthyl or 1,1'-bis(diphenylphosphino)ferrocene. The catalyst may be synthesized by the combination of a suitable source of palladium (0), for example tris(dibenzylidieneacetone)dipalladium (0), with the phosphine ligand, and may either be preformed or formed *in situ* by including the palladium source and phophine ligand in the reaction mixture. The reaction is carried out at a temperature of 0-150 ˚C, and preferably at a temperature of 60-120 ˚C.

Compounds of formula I in which D is sulfur and E is oxygen or $NR^3$ may be prepared from compounds of formula I in which D is oxygen and E is oxygen, or $NR^3$ by reaction with a suitable sulfide in a suitable solvent. The preferred sulfides are phosphorus sulfides, in particular 4-methoxyphenylthionophosphine sulfide dimer ("Lawesson's Reagent"), and diphosphorus pentasulfide. Suitable solvents for the reaction include aryl hydrocarbon solvents, for example toluene or xylene. The reaction is performed at a temperature of 0-200 ˚C, and preferably at a temperature of 50-180 ˚C.

Compounds of formula XVI wherein D is oxygen and E is $NR^3$ may be prepared from compounds of formula XII wherein J is a halogen or $OSO_2CF_3$ substituent at the position of ring $Ar^1$ at which the bond to nitrogen is formed, by reaction with an appropriate amine of formula XIV in which EH is $NHR^3$, or, alternatively, from compounds of formula XVII in which EH is $NHR^3$ by reaction with an appropriate compound of formula IX wherein J is a halogen or $OSO_2CF_3$ substituent at the position of ring $Ar^2$ at which the bond to nitrogen is formed. The reaction may be performed by heating in an inert solvent in the presence of a suitable strong base. Suitable inert solvents include ether solvents, for example tetrahydrofuran, 1,4-dioxane, 1,2-dimethoxyethane, or di(2-methoxyethyl)ether, a hydrocarbon solvent, for example benzene or toluene, or an amide solvent, for example *N,N*-dimethylformamide, or *N*-methyl-2-pyrrolidinone. The preferred solvent is tetrahydrofuran. Suitable strong bases include alkali metal alkoxide or amide bases, for example sodium t-butoxide or potassium t-butoxide, lithium bis(trimethylsilyl)amide, or lithium diisopropylamide. The preferred strong base is sodium t-butoxide. The reaction may require, and is preferably performed in, the presence of an organometallic catalyst. Suitable organometallic catalysts include complexes of palladium (0) with a suitable phosphine ligand, preferably a triarylphosphine ligand, and most preferably a bidentate triarylphosphine ligand. Preferred ligands include 2,2'-bis (diphenylphosphino)-1,1'-binaphthyl or 1,1'-bis(diphenylphosphino)ferrocene. The catalyst may be synthesized by the combination of a suitable source of palladium (0), for example tris(dibenzylideneacetone)dipalladium (0), with the phosphine ligand, and may either be pre-formed or formed *in situ* by including the palladium source and phophine ligand in the reaction mixture. The reaction is carried out at a temperature of 0-150 ˚C, and preferably at a temperature of 60-120 ˚C.

Compounds of formula XVI wherein D is oxygen and E is oxygen or sulfur may be prepared from compounds of formula XII wherein J is a halogen or $OSO_2CF_3$ substituent at the position of ring $Ar^1$ at which the bond to oxygen or sulfur is formed, by reaction with an appropriate compound of formula XIV in which EH is OH or SH, or, alternatively, from compounds of formula XVII in which EH is OH or SH by reaction with an appropriate compound of formula IX wherein J is a halogen or $OSO_2CF_3$ substituent at the position of ring $Ar^2$ at which the bond to oxygen or sulfur is formed. The reaction may be performed by heating in an inert solvent in the presence of a suitable base. The reaction may require, and is preferably performed in, the presence of a catalyst. Suitable inert solvents include ether solvents, for example tetrahydrofuran, 1,4-dioxane, 1,2-dimethoxyethane, or di(2-methoxyethyl)ether, an amide solvent, for example *N,N*-dimethylformamide, or *N*-methyl-2-pyrrolidinone, or a basic heterocyclic aromatic solvent, for example pyridine. The preferred solvent is pyridine. Suitable bases include alkali metal alkoxides, or alkali metal carbonates, for example potassium carbonate. Suitable organometallic catalysts include copper or its salts, preferably copper (I) salts, and most preferably copper (I) iodide. The reaction is carried out at a temperature of 0-150 ˚C, and preferably at a temperature of 100-150 ˚C.

Compounds of formula IX, X, and XII, XIV, and XVII are either commercially available, known in the literature, or may be prepared by methods known to one skilled in the art. In particular, a compound of formula X in which A is a moiety of formula II:

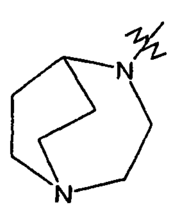

II

may be prepared by the methods described in: J. Gen. Chem. USSR, 1964, 2222-2228, US4,895,543, or EP215650.

It will be appreciated by one skilled in the art that certain optional aromatic substituents in the compounds of the invention may be introduced by employing aromatic substitution reactions, or functional group transformations to modify an existing substituent, or a combination thereof. Such reactions may be effected either prior to or immediately following the processes mentioned above, and are included as part of the process aspect of the invention. The reagents and reaction conditions for such procedures are known in the art. Specific examples of procedures which may be employed include, but are not limited to, electrophilic functionalisation of an aromatic ring, for example via nitration, halogenation, or acylation; transformation of a nitro group to an amino group, for example via reduction, such as by catalytic hydrogenation; acylation, alkylation, sulfonylation of an amino or hydroxyl group; replacement of an amino group by another functional group via conversion to an intermediate diazonium salt followed by nucleophilic or free radical substitution of the diazonium salt; or replacement of a halogen by another functional group, for example via nucleophilic or organometallically-catalysed substitution reactions.

Where necessary, hydroxy, amino, or other reactive groups may be protected using a protecting group as described in the standard text "Protecting groups in Organic Synthesis", 3rd Edition (1999) by Greene and Wuts.

The above described reactions, unless otherwise noted, are usually conducted at a pressure of about one to about three atmospheres, preferably at ambient pressure (about one atmosphere).

Unless otherwise stated, the above described reactions are conducted under an inert atmosphere, preferably under a nitrogen atmosphere.

The compounds of the invention and intermediates may be isolated from their reaction mixtures by standard techniques.

Acid addition salts of the compounds of formula 1 which may be mentioned include salts of mineral acids, for example the hydrochloride and hydrobromide salts; and salts formed with organic acids such as formate, acetate, maleate, benzoate, tartrate, and fumarate salts.

Acid addition salts of compounds of formula I may be formed by reacting the free base or a salt, enantiomer or protected derivative thereof, with one or more equivalents of the appropriate acid. The reaction may be carried out in a solvent or medium in which the salt is insoluble or in a solvent in which the salt is soluble, e.g., water, dioxane, ethanol, tetrahydrofuran or diethyl ether, or a mixture of solvents, which may be removed in vacuum or by freeze drying. The reaction may be a metathetical process or it may be carried out on an ion exchange resin.

The compounds of formula I exist in tautomeric or enantiomeric forms, all of which are included within the scope of the invention. The various optical isomers may be isolated by separation of a racemic mixture of the compounds using conventional techniques, e.g. fractional crystallisation, or chiral HPLC. Alternatively the individual enantiomers may be made by reaction of the appropriate optically active starting materials under reaction conditions which will not cause racemisation.

## Intermediates

A further aspect of the invention relates to novel intermediates. Intermediates of interest are compounds of formula VI in Scheme 1. These intermediates are useful in the synthesis of compounds of formula I, but their use is not limited to the synthesis of such compounds.

Accordingly, there is also provided a compound of formula VI:

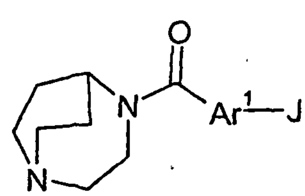

VI

wherein:

- $Ar^1$ is a benzene, furan, or thiophene ring;
- J is halogen, or $OSO_2CF_3$, provided that when $Ar^1$ is a benzene ring, J may only represent halogen or $OSO_2CF_3$ in a position meta or para to the carboxamide group; or an enantiomer thereof or pharmaceutically-acceptable salts thereof.

Particular compounds of this aspect of the invention include:

(1,4-diazabicyclo[3.2.2]non-4-yl)(5-bromofuran-2-yl)methanone;
(1,4-diazabicyclo[3.2.2]non-4-yl)(5-bromothiophen-2-yl)methanone;
(1,4-diazabicyclo[3.2.2]non-4-yl)(3-bromophenyl)methanone;
(1,4-diazabicyclo[3.2.2]non-4-yl)(4-bromophenyl)methanone;
(1,4-diazabicyclo[3.2.2]non-4-yl)(3-iodophenyl)methanone;
(1,4-diazabicyclo[3.2.2]non-4-yl)(4-iodophenyl)methanone;
(1,4-diazabicyclo[3.2.2]non-4-yl)(4-bromothiophen-2-yl)methanone;
(1,4-diazabicyclo[3.2.2]non-4-yl)(5-bromothiophen-3-yl)methanone;
(1,4-diazabicyclo[3.2.2]non-4-yl)(5-bromofuran-2-yl)methanone;
(1,4-diazabicyclo[3.2.2]non-4-yl)(5-bromofuran-2-yl)methanone, and
(1,4-diazabicyclo[3.2.2]non-4-yl)(5-bromofuran-2-yl)methanone;

or enantiomers thereof, or pharmaceutically-acceptable salts thereof.

Intermediate compounds can exist in enantiomeric forms and may be used as purified enantiomers, racemates or mixtures.

**<u>Example 1:</u> (Biphenyl-3-yl)(1,4-diazabicyclo[3.2.2]non-4-yl)methanone**

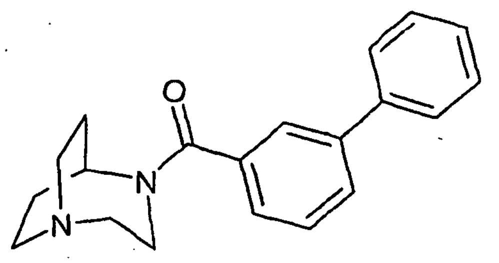

Biphenyl-3-carboxylic acid (52 mg, 0.25 mmol), 1,4-diaza-bicyclo[3.2.2]nonane dihydrochloride (50 mg, 0.25 mmol), 1-hydroxybenzotriazole hydrate (34 mg, 0.25 mmol), O-(benzotriazol-1-yl)-N,N,N',N'-tetramethyluronium tetrafluoroborate (81 mg, 0.25 mL) and diisopropylethylamine (0.17 mL, 125 mg, 1.0 mmol) in dry N,N-dimethylformamide (2 mL) were stirred at ambient temperature for 20 h. The reaction mixture was poured into 1N sodium hydroxide solution and extracted with ethyl acetate (2x). The ethyl acetate layers were combined and washed with water (2x). The solvent was blown off with a stream of nitrogen to yield (biphenyl-3-yl)(1,4-diazabicyclo[3.2.2]non-4-yl)methanone (62 mg, 77%) as a yellow oil. MS (APCI+) 313 [M+1]+; $^1$H-NMR (300 MHz, $CDCl_3$): δ 7.76-7.61 (4H, m), 7.56-7.33 (5H, m), 4.61-4.53 (1H, m), 3.90-3.73 (1H, m), 3.52-3.43 (1H, m), 3.01-2.78 (6H, m), 2.11-1.59 (4H, m).

**<u>Examples 2:</u> (1,4-Diazabicyclo[3.2.2]non-4-yl)(5-phenylfuran-2-yl)methanone**

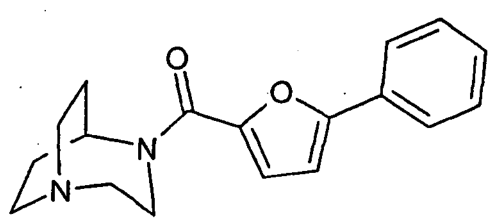

5-Phenylfuran-2-carboxylic acid (49 mg, 0.25 mmol), 1,4-diaza-bicyclo[3.2.2]nonane dihydrochloride (50 mg, 0.25 mmol), 1-hydroxybenzotriazole hydrate (34 mg, 0.25 mmol), O-(benzotriazol-1-yl)-N,N,N',N'-tetramethyluronium tetrafluoroborate (81 mg, 0.25 mL) and diisopropylethylamine (0.17 mL, 125 mg, 1.0 mmol) in dry N,N-dimethylformamide (2 mL) were stirred at ambient temperature for 20 h. The reaction mixture was poured into 1N sodium hydroxide solution and extracted with ethyl acetate (2x). The ethyl acetate layers were combined and washed with water (2x). The solvent was blown off with a stream of nitrogen to yield (1,4-diazabicyclo[3.2.2]non-4-yl)(5-phenylfuran-2-yl)methanone (26 mg,

34%) as a yellow oil.MS (APCI+) 297 [M+I]+. $^1$H-NMR (300 MHz, $CDCl_3$): δ 7.81-7.70 (2H, m), 7.52-7.41 (2H, m), 7.40-7.30 (1H, m), 7.12-7.01 (2H, m), 4.59-4.45 (1H, m), 4.01-3.68 (2H, m), 3.04-2.81 (6H, m), 2.09-1.60 (4H, m).

**Example 3: (1,4-Diazabicyclo[3.2.2]non-4-yl)(5-phenylthiophene-2-yl)methanone**

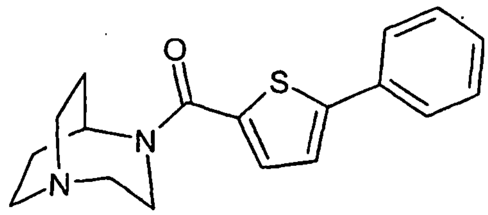

5-Phenylthiophene-2-carboxylic acid (103 mg, 0.50 mmol), 1,4-diazabicyclo[3.2.2]nonane dihydrochloride (100 mg, 0.50 mmol), 1-hydroxybenzotriazole hydrate (68 mg, 0.50 mmol), O-(benzotriazol-1-yl)-N,N,N',N'-tetramethyluronium tetrafluoroborate (161 mg, 0.50 mL) and diisopropylethylamine (0.35 mL, 250 mg, 2.0 mmol) in dry N,N-dimethylformamide (2 mL) were stirred at ambient temperature for 20 h. The reaction mixture was poured into 1N sodium hydroxide solution and extracted with ethyl acetate (2x). The ethyl acetate layers were combined and washed with water (2x). The solvent was blown off with a stream of nitrogen to yield (1,4-diazabicyclo[3.2.2]non-4-yl)(5-phenylthiophene-2-yl)methanone (122 mg, 78%) as a tan oil. MS (APCI+) 313 [M+1]+ $^1$H-NMR (300 MHz, $CDCl_3$): δ 7.74-7.66 (2H, m), 7.53-7.32 (5H, m), 4.53-4.39 (1H, m), 3.89-3.72 (2H, m), 3.01-2.83 (6H, m), 2.06-1.85 (2H, m), 1.82-1.64 (2H, m).

**Example 4: (1,4-Diaza-bicyclo[3.2.2]non-4-yl)-(5-pyridin-2-yl-thiophen-2-yl)-methanone**

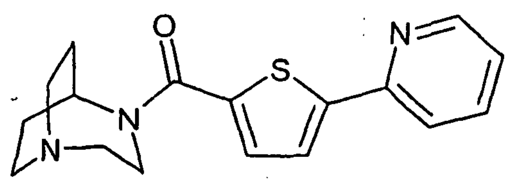

5-(2-Pyridyl)thiophene-2-carboxylic acid (42 mg, 0.25 mmol), 1,4-diazabicyclo[3.2.2]nonane dihydrochloride (50 mg, 0.25 mmol), 1-hydroxybenzotriazole hydrate (34 mg, 0.25 mmol), O-(benzotriazol-1-yl)-N,N,N',N'-tetramethyluronium tetrafluoroborate (81 mg, 0.25 mmol) and diisopropylethylamine (0.17 mL, 129 mg, 1.0 mmol) in dry N,N-dimethylformamide (1.5 mL) were stirred at ambient temperature for 24 h. The reaction mixture was poured into 1N sodium hydroxide solution and extracted with ethyl acetate. The ethyl acetate layer was washed with 1N NaOH (1x), water (4x), brine (1x), and dried over $Na_2SO_4$. After filtration, the solvent was removed *in vacuo* to yield 41 mg of product. The reaction mixture was chromatographed with 100% EtOAc to 90:10 EtOAc:7N $NH_3$/MeOH to give (1,4-diaza-bicyclo [3.2.2]non-4-yl)-(5-pyridin-2-yl-thiophen-2-yl)-methanone (40 mg, 51%) as a colorless oil. MS (APCI+) 314 [M+1]+; $^1$H-NMR (300 MHz, $CDCl_3$): δ 8.58 (1H, d), 7.77-7.65 (2H, m), 7.50 (1H, d), 7.33 (1H, d), 7.21-7.17 (1H, m), 4.68 (1H, s), 3.90 (2H, t), 3.16-2.98 (6H, m), 2.10-2.04 (2H, m), 1.91 (1H, s), 1.86-1.75 (2H, m).

**Example 5: (1,4-Diaza-bicyclo[3.2.2]non-4-yl)-(5-pyridin-3-yl-thiophen-2-yl)-methanone**

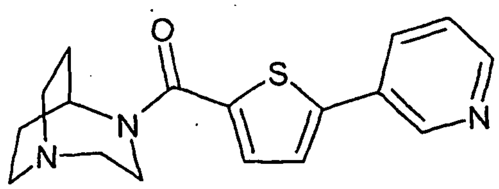

5-Bromothiophene-2-carboxylic acid (104 mg, 0.502 mmol), 1,4-diazabicyclo[3.2.2]nonane dihydrochloride (100 mg, 0.502 mmol), 1-hydroxybenzotriazole hydrate (68 mg, 0.502 mmol), O-(benzotriazol-1-yl)-N,N,N',N'-tetramethyluronium tetrafluoroborate (161 mg, 0.502 mmol) and diisopropylethylamine (0.350 mL, 260 mg, 2.01 mmol) in dry N,N-dimethylformamide (3.0 mL) were stirred at ambient temperature for 24 h. The reaction mixture was poured into 1N sodium hydroxide solution and extracted with ethyl acetate. The ethyl acetate layer was washed with 1N NaOH (1x), water (4x), brine (1x), and dried over $Na_2SO_4$. After filtration, the solvent was removed *in vacuo* to yield (5-Bromothiophen-2-yl)-(1,4-diaza-bicyclo[3.2.2]non-4-yl)-methanone (123 mg, 78%). The product was taken directly into the next reaction without any purification.

A conical microwave vessel was charged with (5-bromo-thiophen-2-yl)-(1,4-diazabicyclo[3.2.2]non-4-yl)-methanone (123mg, 0.390 mmol), 3-pyridylboronic acid (58 mg, 0.468 mmol), dichlorobis(triphenylphosphine)-palladium (II) (2.7 mg, 0.0039 mmol), cesium carbonate (152 mg, 0.468 mmol) and 7:3:2 DME/$H_2O$/EtOH (2.5 mL). The reaction was run in the Smith Synthesizer at 160˚C for 150 seconds. The reaction mixture was filtered through a pad of diatomaceous earth and washed with EtOAc (3x). The combined ethyl acetate layers were washed with $H_2O$, dried over $Na_2SO_4$, filtered and concentrated to give 62 mg of product. The mixture was chromatographed with 100% EtOAc to 90:10 EtOAc: 7N $NH_3$/MeOH to give (1,4-diaza-bicyclo[3.2.2]non-4-yl)-(5-pyridin-3-yl-thiophen-2-yl)-methanone (28 mg, 23%) as a white solid. MS (APCI+) 314 [M+1]+; $^1$H-NMR (300 MHz, $CDCl_3$): δ 8.81 (1H, d), 8.49 (1H, dd), 8.05-7.78 (1H, m), 7.28-7.21 (3H, m), 4.58 (1H, s), 3.85-3.76 (2H, m), 3.09-2.91 (6H, m), 2.03-2.01 (2H, m), 1.80-1.69 (2H, m):

**Example 6: (1,4-Diaza-bycyclo[3.2.2]non-4-yl)-(3-thiophen-2-yl-phenyl)-methanone**

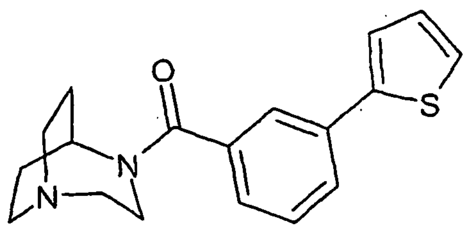

3-Bromobenzoic acid (101 mg, 0.502 mmol), 1,4-diaza-bicyclo[3.2.2]nonane dihydrochloride (100 mg, 0.502 mmol), 1-hydroxybenzotriazole hydrate (68 mg, 0.502 mmol), O-(benzotriazol-1-yl)-N,N,N',N'-tetramethyluronium tetrafluoroborate (161 mg, 0.502 mmol) and diisopropylethylamine (0.350 mL, 260 mg, 2.01 mmol) in dry N,N-dimethylformamide (3.0 mL) were stirred at ambient temperature for 24 h. The reaction mixture was poured into 1N sodium hydroxide solution and extracted with ethyl acetate. The ethyl acetate layer was washed with 1N NaOH (1x), water (4x), brine (1x), and dried over $Na_2SO_4$. After filtration, the solvent was removed *in vacuo* to yield (3-bromo-phenyl)-(1,4-diaza-bicyclo[3.2.2]non-4-yl)-methanone (108 mg, 70%). The product was taken directly into the next reaction without any purification.

A conical microwave vessel was charged with (3-Bromo-phenyl)-(1,4-diazabicyclo[3.2.2]non-4-yl)-methanone (108mg, 0.349 mmol), 2-thiopheneboronic acid (54 mg, 0.419 mmol), dichlorobis(triphenylphosphine)-palladium (II) (2.4 mg, 0.00349 mmol), cesium carbonate (137 mg, 0.419 mmol) and 7:3:2 DME/$H_2O$/EtOH (2.5 mL). The reaction was run in the Smith Synthesizer at 160 ˚C for 150 seconds. The reaction mixture was filtered through a pad of diatomaceous earth and washed with EtOAc (3x). The combined ethyl acetate layers were washed with $H_2O$ dried over $Na_2SO_4$, filtered and concentrated to give 98 mg of product. The mixture prepared on the Gilson reverse phase HPLC to give (1,4-diazabicyclo[3.2.2]non-4-yl)-(3-thiophen-2-yl-phenyl)-methanone (90 mg, 83%) as a colorless oil, TFA salt. MS (APCI+) 313 [M+1]+; $^1$H-NMR (300 MHz, $CDCl_3$): δ 12.04 (1H, s), 7.72 (1H, d), 7.62 (1H, s), 7.47 (1H, t), 7.35-7.29 (3H, m), 7.11 (1H, t), 5.05 (1H, s), 3.93 (2H, s), 3.55-3.52 (6H, m), 2.39 (2H, s), 2.20 (2H, s).

**Example 7: (1,4-Diaza-bicyclo[3.2.2]non-4-yl)-(5-thiophen-2-yl-furan-2-yl)-methanone**

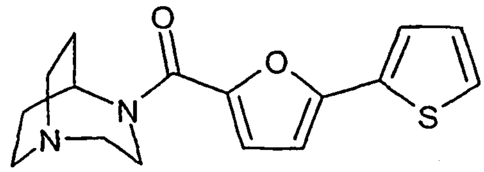

5-Bromo-2-furoic acid (96 mg, 0.502 mmol), 1,4-diaza-bicyclo[3.2.2]nonane dihydrochloride (100 mg, 0.502 mmol), 1-hydroxybenzotriazole hydrate (68 mg, 0.502 mmol), O-(benzotriazol-1-yl)-N,N,N',N'-tetramethyluronium tetrafluoroborate (161 mg, 0.502 mmol) and diisopropylethylamine (0.350 mL, 260 mg, 2.01 mmol) in dry N,N-dimethylformamide (3.0 mL) were stirred at ambient temperature for 24 h. The reaction mixture was poured into 1N sodium hydroxide solution and extracted with ethyl acetate. The ethyl acetate layer was washed with 1N NaOH (1x), water (4x), brine (1x), and dried over $Na_2SO_4$. After filtration, the solvent was removed *in vacuo* to yield (5-bromo-furan-2-yl)-(1,4-diaza-bicyclo[3.2.2]non-4-yl)-methanone (84 mg, 56%). The product was taken directly into the next reaction without any purification.

A conical microwave vessel was charged with (5-bromo-furan-2-yl)-(1,4-diazabicyclo[3.2.2]non-4-yl)-methanone (84 mg, 0.281 mmol), 2-thiopheneboronic acid (43 mg, 0.337 mmol), dichlorobis(triphenylphosphine)-palladium (II) (2.0 mg, 0.00281 mmol), cesium carbonate (110 mg, 0.337 mmol) and 7:3:2 DME/$H_2O$/EtOH (2.5 mL). The reaction was run in the Smith Synthesizer at 160˚C for 150 seconds. The reaction mixture was filtered through a pad of diatomaceous earth and washed with EtOAc (3x). The combined ethyl acetate layers were washed with $H_2O$, dried over $Na_2SO_4$, filtered and concentrated to give 70 mg of product. The mixture prepared on the Gilson reverse phase HPLC to give (1,4-diazabicyclo[3.2.2]non-4-yl)-(5-thiophen-2.yl-furan-2-yl)-methanone (33 mg, 39%) as a colorless oil, TFA salt. MS (APCI+) 303 [M+1]+; $^1$H-NMR (300 MHz, $CDCl_3$): δ 12.55 (1H, s), 7.35 (2H, t), 7.11 (1H, dd), 6.50 (1H, d), 6.54 (1H, s), 5.03-5.01 (1H, m), 4.27 (2H, s), 3.69-3.47 (6H, m), 2.46 (2H, s), 2.29-2.26 (2H, m).

**Example 8: [5-(4-Chlorophenyl)furan-2-yl)(1,4-diaza-bicyclo[3.2.2]non-4-yl)methanone**

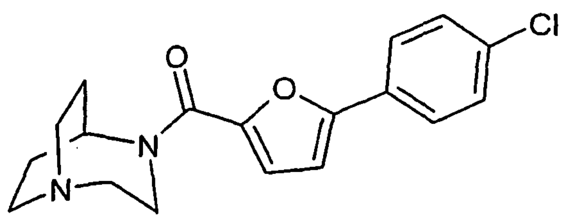

5-(4-Chlorophenyl)furan-2-carboxylic acid (56 mg, 0.25 mmol), 1,4-diazabicyclo[3.2.2]nonane dihydrochloride (50 mg, 0.25 mmol), 1-hydroxybenzotriazole hydrate (34 mg, 0.25 mmol), O-(benzotriazol-1-yl)-N,N,N',N'-tetramethyluronium tetrafluoroborate (81 mg, 0.25 mL) and.diisopropylethylamine (0.17 mL, 125 mg, 1.0 mmol) in dry N,N-dimethylformamide (2 mL) were stirred at ambient temperature for 42 h. The reaction mixture was poured into 1N sodium hydroxide solution and extracted with ethyl acetate. The ethyl acetate layer was washed with 1N NaOH (1x), water (4x), brine (1x) and dried over $Na_2SO_4$. The solvent was removed *in vacuo* to yield [5-(4-chlorophenyl)furan-2-yl](1,4-diazabicyclo[3.2.2]non-4-yl)methanone (76 mg, 92%) as a beige semisolid. MS (APCI+) 331/333 [M+1]+: $^1$H-NMR (300 MHz, $CDCl_3$): δ 7.83-7.74 (2H, d), 7.58-7.49 (2H, d), 7.18-7.H (1H, m), 7.11-7.04 (1H, m), 4.55-4.46 (1H, m), 3.97-3.68 (2H, m), 3.04-2.84 (6H, m), 2.09-1.89 (2H, m), 1.89-1.61 (2H, m).

**Pharmaceutical Compositions**

A further aspect of the invention relates to a pharmaceutical composition for treating or preventing a condition or disorder as exemplified below arising from dysfunction of nicotinic acetylcholine receptor neurotransmission in a mammal, preferably a human, comprising an amount of a compound of formula I, an enantiomer thereof or a pharmaceutically-acceptable salt thereof, effective in treating or preventing such disorder or condition in admixture with an inert pharmaceutically-acceptable diluent or carrier.

For the above-mentioned uses the dosage administered will, of course, vary with the compound employed, the mode of administration and the treatment desired. However, in general, satisfactory results are obtained when the compounds of the invention are administered at a daily dosage of from about 0.1 mg to about 20 mg per kg of animal body weight, preferably given in divided doses 1 to 4 times a day or in sustained release form. For man, the total daily dose is in the range of from 5 mg to 1,400 mg, more preferably from 10 mg to 100 mg, and unit dosage forms suitable for oral administration comprise from 2 mg to 1,400 mg of the compound admixed with a solid or liquid pharmaceutical carrier or diluent.

The compounds of formula I, or an enantiomer thereof, or pharmaceutically-acceptable salts thereof, may be used on their own or in the form of appropriate medicinal preparations for enteral or parenteral administration. According to a further aspect of the invention, there is provided a pharmaceutical composition including preferably less than 80% and more preferably less than 50% by weight of a compound of the invention in admixture with an inert pharmaceutically-acceptable diluent or carrier.

Examples of diluents and carriers are:

- for tablets and dragees: lactose, starch, talc, stearic acid;
- for capsules: tartaric acid or lactose;
- for injectable solutions: water, alcohols, glycerin, vegetable oils;
- for suppositories: natural or hardened oils or waxes.

There is also provided a process for the preparation of such a pharmaceutical composition which comprises mixing the ingredients.

A further aspect of the invention is the use of a compound according to the invention, an enantiomer thereof or a pharmaceutically-acceptable salt thereof, in the manufacture of a medicament for the treatment or prophylaxis of one of the diseases or conditions mentioned herein; and a method of treatment or prophylaxis of one of the above mentioned diseases or conditions, which comprises administering a therapeutically effective amount of a compound according to the invention, or an enantiomer thereof or a pharmaceutically-acceptable salt thereof, to a patient.

Compounds according to the invention are agonists of nicotinic acetylcholine receptors. While not being limited by theory, it is believed that agonists of the $\alpha_7$ nAChR (nicotinic acetylcholine receptor) subtype should be useful in the treatment or prophylaxis of psychotic disorders and intellectual impairment disorders, and have advantages over compounds which are or are also agonists of the $\alpha_4$ nAChR subtype. Therefore, compounds which are selective for the $\alpha_7$ nAChR subtype are preferred. The compounds of the invention are indicated as pharmaceuticals, in particular in the treatment or prophylaxis of psychotic disorders and intellectual impairment disorders. Examples of psychotic disorders include schizophrenia, mania and manic depression, and anxiety. Examples of intellectual impairment disorders include Alzheimer's disease, learning deficit, cognition deficit, attention deficit, Lewy Body Dementia, memory loss, and Attention Deficit Hyperactivity Disorder. The compounds of the invention may also be useful as analgesics in the treatment of pain (including chronic pain) and in the treatment or prophylaxis of Parkinson's disease, Huntington's disease, Tourette's syndrome, and neurodegenerative disorders in which there is loss of cholinergic synapses. The compounds may further be indicated for the treatment or prophylaxis of jetlag, for use in inducing the cessation of smoking, and for the treatment or prophylaxis of nicotine addiction (including that resulting from exposure to products containing nicotine).

It is also believed that compounds according to the invention are useful in the treatment and prophylaxis of ulcerative colitis and irritable bowel disease.

## Pharmacology

The pharmacological activity of the compounds of the invention may be measured in the tests set out below:

Test A - Assay for affinity at $\alpha_7$ nAChR subtype

$^{125}$I-$\alpha$ -Bungarotoxin (BTX) binding to rat hippocampal membranes.

Rat hippocampi were homogenized in 20 volumes of cold homogenisation buffer (HB: concentrations of constituents (mM): tris(hydroxymethyl)aminomethane 50; $MgCl_2$ 1; NaCl 120; KCl 5: pH 7.4). The homogenate was centrifuged for 5 minutes at 1000 xg, the supernatant was saved and the pellet re-extracted. The pooled supernatants were centrifuged for 20 minutes at 12000 xg, washed, and re-suspended in HB. Membranes (30-80 $\mu$g) were incubated with 5 nM [$^{125}$I]$\alpha$-BTX, 1 mg/mL BSA (bovine serum albumin), test drug, and either 2 mM $CaCl_2$ or 0.5 mM EGTA [ethylene glycol-bis($\beta$-aminoethylether)] for 2 hours at 21 °C, and then filtered and washed 4 times over Whatman glass fibre filters

(thickness C) using a Brandel cell harvester. Pre-treating the filters for 3 hours with 1% (BSA/0.01% PEI (polyethyleneimine) in water was critical for low filter blanks (0.07% of total counts per minute). Non-specific binding was described by 100 μM (-)-nicotine, and specific binding was typically 75%.

Test B- Assay for affinity to the $\alpha_4$ nAChR subtype

[$^3$H]-(-)-nicotine binding.

Using a procedure modified from Martino-Barrows and Kellar (Mol Pharm (1987) 31:169-174), rat brain (cortex and hippocampus) was homogenised as in the [$^{125}$I]α-BTX binding assay, centrifuged for 20 minutes at 12,000 xg, washed twice, and then re-suspended in HB containing 100 μM diisopropyl fluorophosphate. After 20 minutes at 4 °C, membranes (approximately 0.5 mg) were incubated with 3 nM [$^3$H]-(-)-nicotine, test drug, 1 μM atropine, and either 2 mM CaCl2 or 0.5 mM EGTA for 1 hour at 4 °C, and then filtered over Whatman glass fibre filters (thickness C) (pre-treated for 1 hour with 0.5% PEI) using a Brandel cell harvester. Non-specific binding was described by 100 μM carbachol, and specific binding was typically 84%.

Binding data analysis for Tests A and B

$IC_{50}$ values and pseudo Hill coefficients ($n_H$) were calculated using the non-linear curve fitting program ALLFIT (DeLean A, Munson P J and Rodbard D (1977) Am. J. Physiol., 235:E97-E102). Saturation curves were fitted to a one site model, using the non-linear regression program ENZFITTER (Leatherbarrow, R.J. (1987)), yielding $K_D$ values of 1.67 and 1.70 nM for the $^{125}$I-α-BTX and [$^3$H]-(-)-nicotine ligands respectively. $K_i$ values were estimated using the general Cheng-Prusoff equation:

$$K_i=[IC_{50}]/((2+([\text{ligand}]/K_D])^n)^{1/n}-1)$$

where a value of n=1 was used whenever $n_H < 1.5$ and a value of n=2 was used when $n_H \geq 1.5$. Samples were assayed in triplicate and were typically ± 5%. $K_i$ values were determined using 6 or more drug concentrations. The compounds of the invention are compounds with binding affinities ($K_i$) of less than 10 μM in either Test A or Test B, indicating that they are expected to have useful therapeutic activity.

The compounds of the invention have the advantage that they may be less toxic, be more efficacious, be longer acting, have a broader range of activity, be more potent, produce fewer side effects, are more easily absorbed or have other useful pharmacological properties.

## Claims

**1.** A compound of formula I:

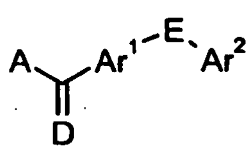

I

wherein:

A is a moiety of formula II:

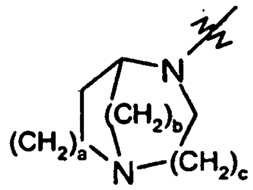

II

D is oxygen or sulfur;
E is a single bond, oxygen, sulfur, or $NR^3$;
$Ar^1$ is a 5- or 6-membered aromatic heterocyclic ring having 1, 2 or 3 heteroatoms selected from nitrogen, oxygen or sulfur where not more than one of said heteroatoms is oxygen or sulfur, or
$Ar^1$ is phenyl;
$Ar^2$ is a 5- or 6-membered aromatic heterocyclic ring having 1, 2 or 3 heteroatoms selected from nitrogen, oxygen or sulfur where not more than one of said heteroatoms is oxygen or sulfur, or
$Ar^2$ is phenyl, or
$Ar^2$ is an 8- or 9-, or 10-membered fused aromatic carbocyclic ring or fused aromatic heterocyclic ring having 1, 2 or 3 heteroatoms selected from nitrogen, oxygen or sulfur where not more than one of said heteroatoms is oxygen or sulfur, or an 8- or 9-, or 10-membered aromatic carbocyclic ring;
the rings $Ar^1$ and $Ar^2$ are substituted with 0, 1, 2 or 3 substituents selected from: halogen, $C_{1-4}$alkyl, $C_{2-4}$alkenyl, $C_{2-4}$alkynyl, CN, $NO_2$, $CF_3NR^1R^2$, $CH_2NR^1R^2$, $OR^2$, $CH_2OR^2$ or $CO_2R^3$;
$R^1$ and $R^2$ at each occurrence are independently selected from hydrogen, $C_{1-4}$alkyl, aryl, heteroaryl, $C(O)R^3$ $C(O)NHR^3$ $CO_2R^3$ or $SO_2R^3$, or
$R^1$ and $R^2$ in combination is $-(CH_2)_jG(CH_2)_k-$ wherein G is oxygen, sulfur, $NR^3$, or a bond;
a, b and c are each 1 or 2;
j is 2, 3 or 4;
k is 0, 1 or 2, and
$R^3$ at each occurrence is independently selected from hydrogen, $C_{1-4}$alkyl, aryl, or heteroaryl;

or a diastereoisomer, enantiomer or pharmaceutically-acceptable salt thereof.

2. A compound according to Claim 1, wherein D is oxygen.

3. A compound according to Claim 2, wherein E is a single bond.

4. A compound according to Claim 2, wherein E is oxygen or $NR^3$.

5. A compound according to Claim 1, wherein A is

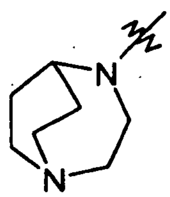

II

or a diastereoisomer, enantiomer or pharmaceutically-acceptable salt thereof.

6. A compound of Claim 1, wherein
$Ar^1$ is a 5- or 6-membered aromatic heterocyclic ring having 1 or 2 heteroatoms selected from nitrogen, oxygen or sulfur where not more than one of said heteroatoms is oxygen or sulfur, or
$Ar^1$ is phenyl,
or a diastereoisomer, enantiomer or pharmaceutically-acceptable salt thereof.

**7.** A compound according to Claim 6 wherein $Ar^1$ is a benzene ring, furan ring or thiophene ring.

**8.** A compound according to Claim 1, wherein
$Ar^2$ is a 5- or 6-membered aromatic heterocyclic ring having 1 or 2 heteroatoms selected from nitrogen, oxygen or sulfur where not more than one of said heteroatoms is oxygen or sulfur, or a phenyl,
or a diastereoisomer, enantiomer or pharmaceutically-acceptable salt thereof.

**9.** A compound according to Claim 8, wherein $Ar^2$ is a benzene ring, furan ring, thiophene ring, or pyridine ring.

**10.** A compound according to Claim 1, wherein
the $-EAr^2$ and the C(=D)A moieties on $Ar^1$ are positioned in a 1,3-relationship relative to each other;
or a diastereoisomer, enantiomer or pharmaceutically-acceptable salt thereof.

**11.** A compound according to Claim 1, wherein $Ar^1$ or $Ar^2$ is substituted with 0 or 1 substituents selected from: halogen, $C_{1-4}$alkyl; $C_{2-4}$alkenyl, $C_{2-4}$alkynyl, CN, $NO_2$, $NR^1R^2$, $CH_2NR^1R^2$, $OR^3$, $CH_2OR^3$, $CO_2R^3$ or $CF_3$;
or a diastereoisomer, enantiomer or pharmaceutically-acceptable salt thereof.

**12.** A compound according to Claim 1, wherein A is a moiety of formula II:

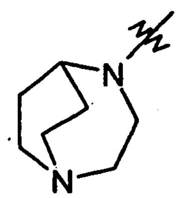

II

D is oxygen;
E is a single bond;
$Ar^1$ is a 5- or 6-membered aromatic heterocyclic ring having 1, 2 or 3 heteroatoms selected from nitrogen, oxygen or sulfur where not more than 1 of said heteroatoms is oxygen or sulfur, or
$Ar^1$ is phenyl
$Ar^2$ is a 5- or 6-membered aromatic heterocyclic ring having 1, 2 or 3 heteroatoms selected from nitrogen, oxygen or sulfur where not more than 1 of said heteroatoms is oxygen or sulfur, or
$Ar^2$ is phenyl,

or a diastereoisomer, enantiomer or pharmaceutically-acceptable salt thereof.

**13.** A compound of Claim 12, wherein $Ar^1$ is a benzene ring, furan ring or thiophene ring.

**14.** A compound according to Claim I, having the groups $-EAr^2$ and -C(=O)A, positioned in a 1,3-relationship relative to each other and wherein $Ar^2$ has 0 or 1 substituents selected from: halogen, $C_{1-4}$alkyl, $C_{2-4}$alkenyl, $C_{2-4}$alkynyl, CN, $NO_2$, $NR^1R^2$, $CH_2NR^1R^2$, $OR^1$, $CH_2OR^1$, $CO_2R^3$ or $CF_3$;
or a diastereoisomer, enantiomer or pharmaceutically-acceptable salt thereof.

**15.** A compound according to Claim 1, selected from:

(1,4-diazabicyclo[3.2.2]non-4-yl)(biphenyl-3-yl)methanone;
(1,4-diazabicyclo[3.2.2]non-4-yl)(3-(2-pyridyl)phenyl)methanone;
(1,4-diazabicyclo[3.2.2]non-4-yl)(3-(3-pyridyl)phenyl)methanone;
(1,4-diazabicyclo[3.2.2]non-4-yl)(3-(4-pyridyl)phenyl)methanone;
(1,4-diazabicyclo[3.2.2]non-4-yl)(3-(furan-2-yl)phenyl)methanone;
(1,4-diazabicyclo[3.2.2]non-4-yl)(3-(furan-3-yl)phenyl)methanone;
(1,4-diazabicyclo[3.2.2]non-4-yl)(3-(thiophen-2-yl)phenyl)methanone;
(1,4-diazabicyclo[3.2.2]non-4-yl)(3-(thiophen-3-yl)phenyl)methanone;
(1,4-diazabicyclo[3.2.2]non-4-yl)(biphenyl-4-yl)methanone;
(1,4-diazabicyclo[3.2.2]non-4-yl)(4-(2-pyridyl)phenyl)methanone;

(1,4-diazabicyclo[3.2.2]non-4-yl)(4-(3-pyridyl)phenyl)methanone;
(1,4-diazabicyclo[3.2.2]non-4-yl)(4-(4-pyridyl)phenyl)methanone;
(1,4-diazabicyclo[3.2.2]non-4-yl)(4-(furan-2-yl)phenyl)methanone;
(1,4-diazabicyclo[3.2.2]non-4-yl)(4-(furan-3-yl)phenyl)methanone;
(1,4-diazabicyclo[3.2.2]non-4-yl)(4-(thiophen-2-yl)phenyl)methanone;
(1,4-diazabicyclo[3.2.2]non-4-yl)(4-(thiophen-3-yl)phenyl)methanone;
(1,4-diazabicyclo[3.2.2]non-4-yl)(5-phenylfuran-2-yl)methanone; (1,4-diazabicyclo[3.2.2]non-4-yl)(5-(2-pyridyl)furan-2-yl)methanone;
(1,4-diazabicyclo[3.2.2]non-4-yl)(5-(3-pyridyl)furan-2-yl)methanone;
(1,4-diazabicyclo[3.2.2]non-4-yl)(5-(4-pyridyl)furan-2-yl)methanone;
(1,4-diazabicyclo[3.2.2]non-4-yl)(5-(furan-2-yl)furan-2-yl)methanone;
(1,4-diazabicyclo[3.2.2]non-4-yl)(5-(furan-3-yl)furan-2-yl)methanone;
(1,4-diazabicyclo[3.2.2]non-4-yl)(5-(thiophen-2-yl)furan-2-yl)methanone;
(1,4-diazabicyclo[3.2.2]non-4-yl)(5-(thiophen-3-yl)furan-2-yl)methanone;
(1,4-diazabicyclo[3.2.2]non-4-yl)(2-phenylthiophen-4-yl)methanone;
(1,4-diazabicyclo[3.2.2]non-4-yl)(2-(2-pyridyl)thiophen-4-yl)methanone;
(1,4-diazabicyclo[3.2.2]non-4-yl)(2-(3-pyridyl)thiophen-4-yl)methanone;
(1,4-diazabicyclo[3.2.2]non-4-yl)(2-(4-pyridyl)thiophen-4-yl)methanone;
(1,4-diazabicyclo[3.2.2]non-4-yl)(2-(furan-2-yl)thiophen-4-yl)methanone;
(1,4-diazabicyclo[3.2.2]non-4-yl)(2-(furan-3-yl)thiophen-4-yl)methanone;
(1,4-diazabicyclo[3.2.2]non-4-yl)(2-(thiophen-2-yl)thiophen-4-yl)methanone;
(1,4-diazabicyclo[3.2.2]non-4-yl)(2-(thiophen-3-yl)thiophen-4-yl)methanone;
(1,4-diazabicyclo[3.2.2]non-4-yl)(4-phenylfuran-2-yl)methanone;
(1,4-diazabicyclo[3.2.2]non-4-yl)(4-(2-pyridyl)furan-2-yl)methanone;
(1,4-diazabicyclo[3.2.2]non-4-yl)(4-(3-pyridyl)furan-2-yl)methanone;
(1,4-diazabicyclo[3.2.2]non-4-yl)(4-(4-pyridyl)furan-2-yl)methanone;
(1,4-diazabicyclo[3.2.2]non-4-yl)(4-(furan-2-yl)furan-2-yl)methanone;
(1,4-diazabicyclo[3.2.2]non-4-yl)(4-(furan-3-yl)furan-2-yl)methanone;
(1,4-diazabicyclo[3.2.2]non-4-yl)(4-(thiophen-2-yl)furan-2-yl)methanone;
(1,4-diazabicyclo[3.2.2]non-4-yl)(4-(thiophen-3-yl)furan-2-yl)methanone;
(1,4-diazabicyclo[3.2.2]non-4-yl)(5-phenylthiophen-2-yl)methanone;
(1,4-diazabicyclo[3.2.2]non-4-yl)(5-(2-pyridyl)thiophen-2-yl)methanone;
(1,4-diazabicyclo[3.2.2]non-4-yl)(5-(3-pyridyl)thiophen-2-yl)methanone;
(1,4-diazabicyclo[3.2.2]non-4-yl)(5-(4-pyridyl)thiophen-2-yl)methanone;
(1,4-diazabicyclo[3.2.2]non-4-yl)(5-(furan-2-yl)thiophen-2-yl)methanone;
(1,4-diazabicyclo[3.2.2]non-4-yl)(5-(furan-3-yl)thiophen-2-yl)methanone;
(1,4-diazabicyclo[3.2.2]non-4-yl)(5-(thiophen-2-yl)thiophen-2-yl)methanone;
(1,4-diazabicyclo[3.2.2]non-4-yl)(5-(thiophen-3-yl)thiophen-2-yl)methanone;
(1,4-diazabicyclo[3.2.2]non-4-yl)(2-phenylfuran-4-yl)methanone;
(1,4-diazabicyclo[3.2.2]non-4-yl)(2-(2-pyridyl)furan-4-yl)methanone;
(1,4-diazabicyclo[3.2.2]non-4-yl)(2-(3-pyridyl)furan-4-yl)methanone;
(1,4-diazabicyclo[3.2.2]non-4-yl)(2-(4-pyridyl)furan-4-yl)methanone;
(1,4-diazabicyclo[3.2.2]non-4-yl)(2-(furan-2-yl)furan-4-yl)methanone;
(1,4-diazabicyclo[3.2.2]non-4-yl)(2-(furan-3-yl)furan-4-yl)methanone;
(1,4-diazabicyclo[3.2.2]non-4-yl)(2-(thiophen-2-yl)furan-4-yl)methanone;
(1,4-diazabicyclo[3.2.2]non-4-yl)(2-(thiophen-3-yl)furan-4-yl)methanone;
(1,4-diazabicyclo[3.2.2]non-4-yl)(4-phenylthiophen-2-yl)methanone;
(1,4-diazabicyclo[3.2.2]non-4-yl)(4-(2-pyridyl)thiophen-2-yl)methanone;
(1,4-diazabicyclo[3.2.2]non-4-yl)(4-(3-pyridyl)thiophen-2-yl)methanone;
(1,4-diazabicyclo[3.2.2]non-4-yl)(4-(4-pyridyl)thiophen-2-yl)methanone;
(1,4-diazabicyclo[3.2.2]non-4-yl)(4-(furan-2-yl)thiophen-2-yl)methanone;
(1,4-diazabicyclo[3.2.2]non-4-yl)(4-(furan-3-yl)thiophen-2-yl)methanone;
(1,4-diazabicyclo[3.2.2]non-4-yl)(4-(thiophen-2-yl)thiophen-2-yl)methanone, or
(1,4-diazabicyclo[3.2.2]non-4-yl)(4-(thiophen-3-yl)thiophen-2-yl)methanone,

or a diastereoisomer, enantiomer or pharmaceutically-acceptable salt thereof.

**16.** A compound according to any one of Claims 1 to 15, for use in therapy.

**17.** A compound according to any one of Claims 1 to 15, for use as a medicament.

**18.** Use of a compound as defined in any one of claims 1 to 15, in the manufacture of a medicament for the treatment or prophylaxis of psychotic disorders, intellectual impairment disorders, human diseases or conditions in which activation of the $\alpha7$ nicotinic receptor is beneficial, Alzheimer's disease, learning deficit, cognition deficit, attention deficit, memory loss, Lewy Body Dementia, Attention Deficit Hyperactivity Disorder, anxiety, schizophrenia, mania or manic depression, Parkinson's disease, Huntington's disease, Tourette's syndrome, neurodegenerative disorders in which there is loss of cholinergic synapse, jetlag, cessation of smoking, nicotine addiction including that resulting from exposure to products containing nicotine, pain, ulcerative colitis or irritable bowel syndrome.

**19.** A pharmaceutical composition comprising a compound of formula I, as defined in any one of claims 1 to 15, together with at least one pharmaceutically-acceptable excipient or diluent.

**20.** A process for the preparation of a compound of formula I, as defined in any one of claims 1 to 15, which comprises:

reacting a compound of formula VI:

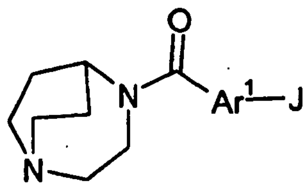

VI

wherein J represents halogen, or $OSO_2CF_3$ substituent at the position of ring $Ar^1$ at which the bond to ring $Ar^2$ is formed with a organometallic compound of formula VII;

$Ar^2$-M VII

in the presence of a organometallic catalyst and solvent.

**21.** A compound of formula VI:

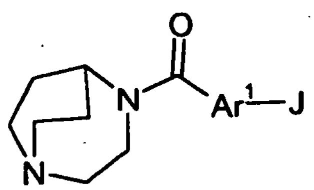

VI

wherein:

$Ar^1$ is a benzene, furan, or thiophene ring;
J is halogen, or $OSO_2CF_3$, provided that when $Ar^1$ is a benzene ring, J may only represent halogen or $OSO_2CF_3$ in a position meta or para to the carboxamide group; or an enantiomer thereof or pharmaceutically-acceptable salts thereof.

**22.** A compound according to Claim 21, selected from:

(1,4-diazabicyclo[3.2.2]non-4-yl)(5-bromofuran-2-yl)methanone;
(1,4-diazabicyclo[3.2.2]non-4-yl)(5-bromothiophen-2-yl)methanone;
(1,4-diazabicyclo[3.2.2]non-4-yl)(3-bromophenyl)methanone;
(1,4-diazabicyclo[3.2.2]non-4-yl)(4-bromophenyl)methanone;
(1,4-diazabicyclo[3.2.2]non-4-yl)(3-iodophenyl)methanone;

(1,4-diazabicyclo[3.2.2]non-4-yl)(4-iodophenyl)methanone;
(1,4-diazabicyclo[3.2.2]non-4-yl)(4-bromothiophen-2-yl)methanone; and
(1,4-diazabicyclo[3.2.2]non-4-yl)(5-bromothiophen-3-yl)methanone;

or an enantiomer thereof, or a pharmaceutically-acceptable salt thereof.

## Patentansprüche

**1.** Verbindung der Formel I:

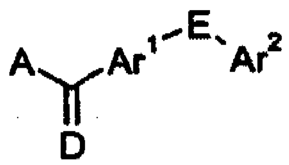

I

worin:

A für eine Gruppierung der Formel II:

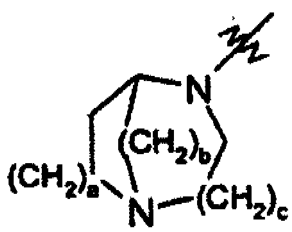

II

steht;
D für Sauerstoff oder Schwefel steht;
E für eine Einfachbindung, Sauerstoff, Schwefel oder $NR^3$ steht;
$Ar^1$ für einen 5- oder 6-gliedrigen aromatischen heterocyclischen Ring mit 1, 2 oder 3 unter Stickstoff, Sauerstoff oder Schwefel ausgewählten Heteroatomen, wobei nicht mehr als eines der Heteroatome Sauerstoff oder Schwefel ist, steht oder
$Ar^1$ für Phenyl steht;
$Ar^2$ für einen 5- oder 6-gliedrigen aromatischen heterocyclischen Ring mit 1, 2 oder 3 unter Stickstoff, Sauerstoff oder Schwefel ausgewählten Heteroatomen, wobei nicht mehr als eines der Heteroatome Sauerstoff oder Schwefel ist, steht oder
$Ar^2$ für Phenyl steht oder
$Ar^2$ für einen 8-, 9- oder 10-gliedrigen kondensierten aromatischen carbocyclischen Ring oder kondensierten aromatischen heterocyclischen Ring mit 1, 2 oder 3 unter Stickstoff, Sauerstoff oder Schwefel ausgewählten Heteroatomen, wobei nicht mehr als eines der Heteroatome Sauerstoff oder Schwefel ist, oder einen 8-, 9- oder 10-gliedrigen aromatischen carbocyclischen Ring steht;
die Ringe $Ar^1$ und $Ar^2$ durch 0, 1, 2 oder 3 unter: Halogen, $C_1$-$C_4$-Alkyl, $C_2$-$C_4$-Alkenyl, $C_2$-$C_4$-Alkinyl, CN, $NO_2$, $CF_3$, $NR^1R^2$, $CH_2NR^1R^2$, $OR^2$, $CH_2OR^2$ oder $CO_2R^3$ ausgewählte Substituenten substituiert sind;
$R^1$ und $R^2$ jeweils unabhängig voneinander unter Wasserstoff, $C_1$-$C_4$-Alkyl, Aryl, Heteroaryl, $C(O)R^3$, $C(O)NHR^3$, $CO_2R^3$ oder $SO_2R^3$ ausgewählt sind oder
$R^1$ und $R^2$ gemeinsam für $-(CH_2)_jG(CH_2)_k-$, worin G Sauerstoff, Schwefel, $NR^3$ oder eine Bindung bedeutet, stehen;
a, b und c jeweils für 1 oder 2 stehen;
j für 2, 3 oder 4 steht;
k für 0, 1 oder 2 steht und

$R^3$ jeweils unabhängig voneinander unter Wasserstoff, $C_1$-$C_4$-Alkyl, Aryl oder Heteroaryl ausgewählt ist;

oder ein Diastereoisomer, Enantiomer oder pharmazeutisch annehmbares Salz davon.

**2.** Verbindung nach Anspruch 1, worin D für Sauerstoff steht.

**3.** Verbindung nach Anspruch 2, worin E für eine Einfachbindung steht.

**4.** Verbindung nach Anspruch 2, worin E für Sauerstoff oder $NR^3$ steht.

**5.** Verbindung nach Anspruch 1, worin A für

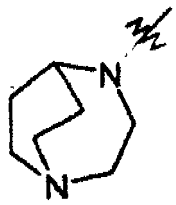

II

steht,
oder ein Diastereoisomer, Enantiomer oder pharmazeutisch annehmbares Salz davon.

**6.** Verbindung nach Anspruch 1, worin
$Ar^1$ für einen 5- oder 6-gliedrigen aromatischen heterocyclischen Ring mit 1 oder 2 unter Stickstoff, Sauerstoff und Schwefel ausgewählten Heteroatomen, wobei nicht mehr als eines der Heteroatome Sauerstoff oder Schwefel ist, steht oder
$Ar^1$ für Phenyl steht,
oder ein Diastereoisomer, Enantiomer oder pharmazeutisch annehmbares Salz davon.

**7.** Verbindung nach Anspruch 6, worin $Ar^1$ für einen Benzolring, Furanring oder Thiophenring steht.

**8.** Verbindung nach Anspruch 1, worin
$Ar^2$ für einen 5- oder 6-gliedrigen aromatischen heterocyclischen Ring mit 1 oder 2 unter Stickstoff, Sauerstoff und Schwefel ausgewählten Heteroatomen, wobei nicht mehr als eines der Heteroatome Sauerstoff oder Schwefel ist, oder Phenyl steht,
oder ein Diastereoisomer, Enantiomer oder pharmazeutisch annehmbares Salz davon.

**9.** Verbindung nach Anspruch 8, worin $Ar^2$ für einen Benzolring, Furanring, Thiophenring oder Pyridinring steht.

**10.** Verbindung nach Anspruch 1, worin
die Gruppierungen $-EAr^2$ und C(=D)A an $Ar^1$ in 1,3-Beziehung zueinander stehen;
oder ein Diastereoisomer, Enantiomer oder pharmazeutisch annehmbares Salz davon.

**11.** Verbindung nach Anspruch 1, worin $Ar^1$ oder $Ar^2$ durch 0 oder 1 unter Halogen, $C_1$-$C_4$-Alkyl, $C_2$-$C_4$-Alkenyl, $C_2$-$C_4$-Alkinyl, CN, $NO_2$, $NR^1R^2$, $CH_2NR^1R^2$, $OR^3$, $CH_2OR^3$, $CO_2R^3$ oder $CF_3$ ausgewählte Substituenten substituiert sind;
oder ein Diastereoisomer, Enantiomer oder pharmazeutisch annehmbares Salz davon.

**12.** Verbindung nach Anspruch 1, worin A für eine Gruppierung der Formel II:

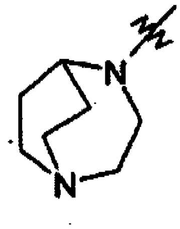

II

steht;

D für Sauerstoff steht;
E für eine Einfachbindung steht;
$Ar^1$ für einen 5- oder 6-gliedrigen aromatischen heterocyclischen Ring mit 1, 2 oder 3 unter Stickstoff, Sauerstoff oder Schwefel ausgewählten Heteroatomen, wobei nicht mehr als eines der Heteroatome Sauerstoff oder Schwefel ist, steht oder
$Ar^1$ für Phenyl steht;
$Ar^2$ für einen 5- oder 6-gliedrigen aromatischen heterocyclischen Ring mit 1, 2 oder 3 unter Stickstoff, Sauerstoff oder Schwefel ausgewählten Heteroatomen, wobei nicht mehr als eines der Heteroatome Sauerstoff oder Schwefel ist, steht oder
$Ar^2$ für Phenyl steht,

oder ein Diastereoisomer, Enantiomer oder pharmazeutisch annehmbares Salz davon.

**13.** Verbindung nach Anspruch 12, worin $Ar^1$ für einen Benzolring, Furanring oder Thiophenring steht.

**14.** Verbindung nach Anspruch 1, worin die Gruppen $-EAr^2$ und -C(=O)A in 1,3-Beziehung zueinander stehen und $Ar^2$ 0 oder 1 unter Halogen, $C_1$-$C_4$-Alkyl, $C_2$-$C_4$-Alkenyl, $C_2$-$C_4$-Alkinyl, CN, $NO_2$, $NR^1R^2$, $CH_2NR^1R^2$, $OR^1$, $CH_2OR^1$, $CO_2R^3$ oder $CF_3$ ausgewählte Substituenten aufweist;
oder ein Diastereoisomer, Enantiomer oder pharmazeutisch annehmbares Salz davon.

**15.** Verbindung nach Anspruch 1, ausgewählt unter:

(1,4-Diazabicyclo[3.2.2]non-4-yl)(biphenyl-3-yl)-methanon;
(1,4-Diazabicyclo[3.2.2]non-4-yl)(3-(2-pyridyl)-phenyl)methanon;
(1,4-Diazabicyclo[3.2.2]non-4-yl)(3-(3-pyridyl)-phenyl)methanon;
(1,4-Diazabicyclo[3.2.2]non-4-yl)(3-(4-pyridyl)-phenyl)methanon;
(1,4-Diazabicyclo[3.2.2]non-4-yl)(3-(furan-2-yl)-phenyl)methanon;
(1,4-Diazabicyclo[3.2.2]non-4-yl)(3-(furan-3-yl)-phenyl)methanon;
(1,4-Diazabicyclo[3.2.2]non-4-yl)(3-(thiophen-2-yl)phenyl)methanon;
(1,4-Diazabicyclo[3.2.2]non-4-yl)(3-(thiophen-3-yl)phenyl)methanon;
(1,4-Diazabicyclo[3.2.2]non-4-yl)(biphenyl-4-yl)-methanon;
(1,4-Diazabicyclo[3.2.2]non-4-yl)(4-(2-pyridyl)-phenyl)methanon;
(1,4-Diazabicyclo[3.2.2]non-4-yl)(4-(3-pyridyl)-phenyl)methanon;
(1,4-Diazabicyclo[3.2.2]non-4-yl)(4-(4-pyridyl)-phenyl)methanon;
(1,4-Diazabicyclo[3.2.2]non-4-yl)(4-(furan-2-yl)-phenyl)methanon;
(1,4-Diazabicyclo[3.2.2]non-4-yl)(4-(furan-3-yl)-phenyl)methanon;
(1,4-Diazabicyclo[3.2.2]non-4-yl)(4-(thiophen-2-yl)phenyl)methanon;
(1,4-Diazabicyclo[3.2.2]non-4-yl)(4-(thiophen-3-yl)phenyl)methanon;
(1,4-Diazabicyclo[3.2.2]non-4-yl)(5-phenylfuran-2-yl)methanon;
(1,4-Diazabicyclo[3.2.2]non-4-yl)(5-(2-pyridyl)-furan-2-yl)methanon;
(1,4-Diazabicyclo[3.2.2]non-4-yl)(5-(3-pyridyl)-furan-2-yl)methanon;
(1,4-Diazabicyclo[3.2.2]non-4-yl)(5-(4-pyridyl)-furan-2-yl)methanon;
(1,4-Diazabicyclo[3.2.2]non-4-yl)(5-(furan-2-yl)-furan-2-yl)methanon;
(1,4-Diazabicyclo[3.2.2]non-4-yl)(5-(furan-3-yl)-furan-2-yl)methanon;
(1,4-Diazabicyclo[3.2.2]non-4-yl)(5-(thiophen-2-yl)furan-2-yl)methanon;
(1,4-Diazabicyclo[3.2.2]non-4-yl)(5-(thiophen-3-yl)furan-2-yl)methanon;

(1,4-Diazabicyclo[3.2.2]non-4-yl)(2-phenyl-thiophen-4-yl)methanon;
(1,4-Diazabicyclo[3.2.2]non-4-yl)(2-(2-pyridyl)-thiophen-4-yl)methanon;
(1,4-Diazabicyclo[3.2.2]non-4-yl)(2-(3-pyridyl)-thiophen-4-yl)methanon;
(1,4-Diazabicyclo[3.2.2]non-4-yl)(2-(4-pyridyl)-thiophen-4-yl)methanon;
(1,4-Diazabicyclo[3.2.2]non-4-yl)(2-(furan-2-yl)-thiophen-4-yl)methanon;
(1,4-Diazabicyclo[3.2.2]non-4-yl)(2-(furan-3-yl)-thiophen-4-yl)methanon;
(1,4-Diazabicyclo[3.2.2]non-4-yl)(2-(thiophen-2-yl)thiophen-4-yl)methanon;
(1,4-Diazabicyclo[3.2.2]non-4-yl)(2-(thiophen-3-yl)thiophen-4-yl)methanon;
(1,4-Diazabicyclo[3.2.2]non-4-yl)(4-phenylfuran-2-yl)methanon;
(1,4-Diazabicyclo[3.2.2]non-4-yl)(4-(2-pyridyl)-furan-2-yl)methanon;
(1,4-Diazabicyclo[3.2.2]non-4-yl)(4-(3-pyridyl)-furan-2-yl)methanon;
(1,4-Diazabicyclo[3.2.2]non-4-yl)(4-(4-pyridyl)-furan-2-yl)methanon;
(1,4-Diazabicyclo[3.2.2]non-4-yl)(4-(furan-2-yl)-furan-2-yl)methanon;
(1,4-Diazabicyclo[3.2.2]non-4-yl)(4-(furan-3-yl)-furan-2-yl)methanon;
(1,4-Diazabicyclo[3.2.2]non-4-yl)(4-(thiophen-2-yl)furan-2-yl)methanon;
(1,4-Diazabicyclo[3.2.2]non-4-yl)(4-(thiophen-3-yl)furan-2-yl)methanon;
(1,4-Diazabicyclo[3.2.2]non-4-yl)(5-phenyl-thiophen-2-yl)methanon;
(1,4-Diazabicyclo[3.2.2]non-4-yl)(5-(2-pyridyl)-thiophen-2-yl)methanon;
(1,4-Diazabicyclo[3.2.2]non-4-yl)(5-(3-pyridyl)-thiophen-2-yl)methanon;
(1,4-Diazabicyclo[3.2.2]non-4-yl)(5-(4-pyridyl)-thiophen-2-yl)methanon;
(1,4-Diazabicyclo[3.2.2]non-4-yl)(5-(furan-2-yl)-thiophen-2-yl)methanon;
(1,4-Diazabicyclo[3.2.2]non-4-yl)(5-(furan-3-yl)-thiophen-2-yl)methanon;
(1,4-Diazabicyclo[3.2.2]non-4-yl)(5-(thiophen-2-yl)thiophen-2-yl)methanon;
(1,4-Diazabicyclo[3.2.2]non-4-yl)(5-(thiophen-3-yl)thiophen-2-yl)methanon;
(1,4-Diazabicyclo[3.2.2]non-4-yl)(2-phenylfuran-4-yl)methanon;
(1,4-Diazabicyclo[3.2.2]non-4-yl)(2-(2-pyridyl)-furan-4-yl)methanon;
(1,4-Diazabicyclo[3.2.2]non-4-yl)(2-(3-pyridyl)-furan-4-yl)methanon;
(1,4-Diazabicyclo[3.2.2]non-4-yl)(2-(4-pyridyl)-furan-4-yl)methanon;
(1,4-Diazabicyclo[3.2.2]non-4-yl)(2-(furan-2-yl)-furan-4-yl)methanon;
(1,4-Diazabicyclo[3.2.2]non-4-yl)(2-(furan-3-yl)-furan-4-yl)methanon;
(1,4-Diazabicyclo[3.2.2]non-4-yl)(2-(thiophen-2-yl)furan-4-yl)methanon;
(1,4-Diazabicyclo[3.2.2]non-4-yl)(2-(thiophen-3-yl)furan-4-yl)methanon;
(1,4-Diazabicyclo[3.2.2]non-4-yl)(4-phenyl-thiophen-2-yl)methanon;
(1,4-Diazabicyclo[3.2.2]non-4-yl)(4-(2-pyridyl)-thiophen-2-yl)methanon;
(1,4-Diazabicyclo[3.2.2]non-4-yl)(4-(3-pyridyl)-thiophen-2-yl)methanon;
(1,4-Diazabicyclo[3.2.2]non-4-yl)(4-(4-pyridyl)-thiophen-2-yl)methanon;
(1,4-Diazabicyclo[3.2.2]non-4-yl)(4-(furan-2-yl)-thiophen-2-yl)methanon;
(1,4-Diazabicyclo[3.2.2]non-4-yl)(4-(furan-3-yl)-thiophen-2-yl)methanon;
(1,4-Diazabicyclo[3.2.2]non-4-yl)(4-(thiophen-2-yl)thiophen-2-yl)methanon oder
(1,4-Diazabicyclo[3.2.2]non-4-yl)(4-(thiophen-3-yl)thiophen-2-yl)methanon,

oder ein Diastereoisomer, Enantiomer oder pharmazeutisch annehmbares Salz davon.

**16.** Verbindung nach einem der Ansprüche 1 bis 15 zur Verwendung bei der Therapie.

**17.** Verbindung nach einem der Ansprüche 1 bis 15 zur Verwendung als Arzneimittel.

**18.** Verwendung einer Verbindung nach einem der Ansprüche 1 bis 15 bei der Herstellung eines Arzneimittels zur Behandlung oder Prophylaxe von psychotischen Störungen, Intelligenzstörungen, Krankheiten oder Zuständen des Menschen, bei denen die Aktivierung des nicotinischen Rezeptors $\alpha 7$ vorteilhaft ist, Alzheimer-Krankheit, Lernschwäche, Denkschwäche, Konzentrationsstörungen, Gedächtnisschwund, Lewy-Körperchen-Demenz, hyperkinetischem Syndrom, Angst, Schizophrenie, Manie oder manischer Depression, Parkinson-Krankheit, Chorea Huntington, Tourette-Syndrom, neurodegenerativen Erkrankungen mit Verlust cholinerger Synapsen, Jet-lag, Raucherentwöhnung, Nicotinabhängigkeit einschließlich der sich aus der Exposition gegenüber nicotinhaltigen Produkten ergebenden Abhängigkeit, Schmerzen, Colitis ulcerosa oder Reizkolon.

**19.** Pharmazeutische Zusammensetzung, enthaltend eine Verbindung der Formel (I) nach einem der Ansprüche 1 bis 15 zusammen mit mindestens einem pharmazeutisch annehmbaren Hilfsstoff oder Verdünnungsmittel.

**20.** Verfahren zur Herstellung einer Verbindung der Formel I gemäß einem der Ansprüche 1 bis 15, bei dem man:

eine Verbindung der Formel VI:

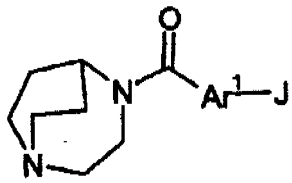

VI

worin J für einen Halogen- oder $OSO_2CF_3$-Substituenten an der Position des Rings $Ar^1$, an der die Bindung zum Ring $Ar^2$ geknüpft wird, steht, in Gegenwart eines metallorganischen Katalysators und eines Lösungsmittels mit einer metallorganischen Verbindung der Formel VII

$Ar^2$-M VII

umsetzt.

**21.** Verbindung der Formel VI:

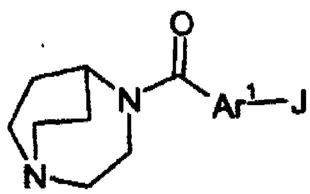

VI

worin:

$Ar^1$ für einen Benzol-, Furan- oder Thiophenring steht;
J für Halogen oder $OSO_2CF_3$ steht, mit der Maßgabe, daß dann, wenn $Ar^1$ für einen Benzolring steht, J nur für Halogen oder $OSO_2CF_3$ in meta- oder para-Position zur Carboxamidgruppe stehen kann; oder ein Enantiomer davon oder pharmazeutisch annehmbare Salze davon.

**22.** Verbindung nach Anspruch 21, ausgewählt unter:

(1,4-Diazabicyclo[3.2.2]non-4-yl)(5-bromfuran-2-yl)methanon;
(1,4-Diazabicyclo[3.2.2]non-4-yl)(5-bromthiophen-2-yl)methanon;
(1,4-Diazabicyclo[3.2.2]non-4-yl)(3-bromphenyl)-methanon;
(1,4-Diazabicyclo[3.2.2]non-4-yl)(4-bromphenyl)-methanon;
(1,4-Diazabicyclo[3.2.2]non-4-yl)(3-iodphenyl)-methanon;
(1,4-Diazabicyclo[3.2.2]non-4-yl)(4-iodphenyl)-methanon;
(1,4-Diazabicyclo[3.2.2]non-4-yl)(4-bromthiophen-2-yl)methanon; und
(1,4-Diazabicyclo[3.2.2]non-4-yl)(5-bromthiophen-3-yl)methanon;

oder ein Enantiomer davon oder pharmazeutisch annehmbare Salze davon.

**Revendications**

**1.** Composé de formule I :

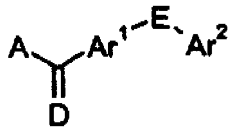

I

dans laquelle :

A est un motif de formule II :

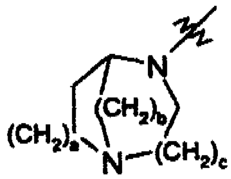

II

D est oxygène ou soufre ;
E est une simple liaison, oxygène, soufre ou $NR^3$ ;
$Ar^1$ est un cycle hétérocyclique aromatique à 5 ou 6 chaînons ayant 1, 2 ou 3 hétéroatomes choisis parmi azote, oxygène ou soufre, où pas plus d'un desdits hétéroatomes est oxygène ou soufre, ou
$Ar^1$ est phényle ;
$Ar^2$ est un cycle hétérocyclique aromatique à 5 ou 6 chaînons ayant 1, 2 ou 3 hétéroatomes choisis parmi azote, oxygène ou soufre, où pas plus d'un desdits hétéroatomes est oxygène ou soufre, ou
$Ar^2$ est phényle ou
$Ar^2$ est un cycle carbocyclique aromatique condensé à 8, 9 ou 10 chaînons, ou un cycle hétérocyclique aromatique condensé ayant 1, 2 ou 3 hétéroatomes choisis parmi azote, oxygène ou soufre, où pas plus d'un desdits hétéroatomes est oxygène ou soufre, ou un cycle carbocyclique aromatique à 8, 9 ou 10 chaînons ;
les cycles $Ar^1$ et $Ar^2$ sont substitués par 0, 1, 2 ou 3 substituants choisis parmi : halogène, $C_{1-4}$alkyle, $C_{2-4}$alcényle, $C_{2-4}$alcynyle, CN, $NO_2$, $CF_3$, $NR^1R^2$, $CH_2NR^1R^2$, $OR^2$, $CH_2OR^2$ ou $CO_2R^3$ ;
$R^1$ et $R^2$ sont, à chaque apparition, choisis indépendamment parmi hydrogène, $C_{1-4}$alkyle, aryle, hétéroaryle, $C(O)R^3$, $C(O)NHR^3$, $CO_2R^3$ ou $SO_2R^3$, ou
$R^1$ et $R^2$, en combinaison, sont $-(CH_2)_jG(CH_2)_k-$, où G est oxygène, soufre, $NR^3$ ou une liaison ;
a, b et c sont chacun 1 ou 2 ;
j est 2, 3 ou 4 ;
k est 0, 1 ou 2, et
$R^3$ est, à chaque apparition, choisi indépendamment parmi hydrogène, $C_{1-4}$alkyle, aryle ou hétéroaryle ;

ou un diastéréoisomère, un énantiomère ou un sel pharmaceutiquement acceptable de celui-ci.

2. Composé selon la revendication 1, **caractérisé en ce que** D est oxygène.

3. Composé selon la revendication 2, **caractérisé en ce que** E est une simple liaison.

4. Composé selon la revendication 2, **caractérisé en ce que** E est oxygène ou $NR^3$.

5. Composé selon la revendication 1, **caractérisé en ce que** A est

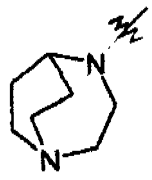

II

ou un diastéréoisomère, un énantiomère ou un sel pharmaceutiquement acceptable de celui-ci.

6. Composé selon la revendication 1, **caractérisé en ce que** :

   $Ar^1$ est un cycle hétérocyclique aromatique à 5 ou 6 chaînons ayant 1 ou 2 hétéroatomes choisis parmi azote, oxygène ou soufre, où pas plus d'un desdits hétéroatomes est oxygène ou soufre, ou
   $Ar^1$ est phényle,

   ou un diastéréoisomère, un énantiomère ou un sel pharmaceutiquement acceptable de celui-ci.

7. Composé selon la revendication 6, **caractérisé en ce que** $Ar^1$ est un cycle benzène, un cycle furane ou un cycle thiophène.

8. Composé selon la revendication 1, **caractérisé en ce que** :

   $Ar^2$ est un cycle hétérocyclique aromatique à 5 ou 6 chaînons ayant 1 ou 2 hétéroatomes choisis parmi azote, oxygène ou soufre, où pas plus d'un desdits hétéroatomes est oxygène ou soufre, ou un phényle,
   ou un diastéréoisomère, un énantiomère ou un sel pharmaceutiquement acceptable de celui-ci.

9. Composé selon la revendication 8, **caractérisé en ce que** $Ar^2$ est un cycle benzène, un cycle furane, un cycle thiophène ou un cycle pyridine.

10. Composé selon la revendication 1, **caractérisé en ce que** :

    les motifs $-EAr^2$ et C(=D)A sur $Ar^1$ sont positionnés dans une relation 1,3 l'un par rapport à l'autre ;
    ou un diastéréoisomère, un énantiomère ou un sel pharmaceutiquement acceptable de celui-ci.

11. Composé selon la revendication 1, **caractérisé en ce que** $Ar^1$ ou $Ar^2$ est substitué par 0 ou 1 substituant choisi parmi halogène, $C_{1-4}$alkyle, $C_{2-4}$alcényle, $C_{2-4}$alcynyle, CN, $NO_2$, $NR^1R^2$, $CH_2NR^1R^2$, $OR^3$, $CH_2OR^3$, $CO_2R^3$ ou $CF_3$ ;
    ou un diastéréoisomère, un énantiomère ou un sel pharmaceutiquement acceptable de celui-ci.

12. Composé selon la revendication 1, **caractérisé en ce que** A est un motif de formule II :

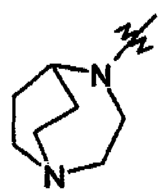

II

    D est oxygène ;
    E est une simple liaison ;
    $Ar^1$ est un cycle hétérocyclique aromatique à 5 ou 6 chaînons ayant 1, 2 ou 3 hétéroatomes choisis parmi azote, oxygène ou soufre, où pas plus d'1 desdits hétéroatomes est oxygène ou soufre, ou
    $Ar^1$ est phényle ;
    $Ar^2$ est un cycle hétérocyclique aromatique à 5 ou 6 chaînons ayant 1, 2 ou 3 hétéroatomes choisis parmi azote, oxygène ou soufre, où pas plus d'1 desdits hétéroatomes est oxygène ou soufre, ou

$Ar^2$ est phényle,

ou un diastéréoisomère, un énantiomère ou un sel pharmaceutiquement acceptable de celui-ci.

**13.** Composé de la revendication 12, **caractérisé en ce que** $Ar^1$ est un cycle benzène, un cycle furane ou un cycle thiophène.

**14.** Composé selon la revendication 1 ayant les groupements $-EAr^2$ et -C(=O)A positionnés dans une relation 1,3 l'un par rapport à l'autre, et où $Ar^2$ a 0 ou 1 substituant choisi parmi : halogène, $C_{1-4}$-alkyle, $C_2$-$_4$alcényle, $C_{2-4}$alcynyle, CN, $NO_2$, $NR^1R^2$, $CH_2NR^1R^2$, $OR^1$, $CH_2OR^1$, $CO_2R^3$ ou $CF_3$ ;
ou un diastéréoisomère, un énantiomère ou un sel pharmaceutiquement acceptable de celui-ci.

**15.** Composé selon la revendication 1 choisi parmi :

la (1,4-diazabicyclo[3,2,2]non-4-yl)(biphényl-3-yl)-méthanone ;
la (1,4-diazabicyclo[3,2,2]non-4-yl)(3-(2-pyridyl)-phényl)méthanone ;
la (1,4-diazabicyclo[3,2,2]non-4-yl)(3-(3-pyridyl)-phényl)méthanone ;
la (1,4-diazabicyclo[3,2,2]non-4-yl)(3-(4-pyridyl)-phényl)méthanone ;
la (1,4-diazabicyclo[3,2,2]non-4-yl)(3-(furan-2-yl)-phényl)méthanone ;
la (1,4-diazabicyclo[3,2,2]non-4-yl)(3-(furan-3-yl)-phényl)méthanone ;
la (1,4-diazabicyclo[3,2,2]non-4-yl)(3-(thiophén-2-yl)phényl)méthanone ;
la (1,4-diazabicyclo[3,2,2]non-4-yl)(3-(thiophén-3-yl)phényl)méthanone ;
la (1,4-diazabicyclo[3,2,2]non-4-yl)(biphényl-4-yl)-méthanone ;
la (1,4-diazabicyclo[3,2,2]non-4-yl)(4-(2-pyridyl)-phényl)méthanone ;
la (1,4-diazabicyclo[3,2,2]non-4-yl)(4-(3-pyridyl)-phényl)méthanone ;
la (1,4-diazabicyclo[3,2,2]non-4-yl)(4-(4-pyridyl)-phényl)méthanone ;
la (1,4-diazabicyclo[3,2,2]non-4-yl)(4-(furan-2-yl)-phényl)méthanone ;
la (1,4-diazabicyclo[3,2,2]non-4-yl)(4-(furan-3-yl)-phényl)méthanone ;
la (1,4-diazabicyclo[3,2,2]non-4-yl)(4-(thiophén-2-yl)phényl)méthanone ;
la (1,4-diazabicyclo[3,2,2]non-4-yl)(4-(thiophén-3-yl)phényl)méthanone ;
la (1,4-diazabicyclo[3,2,2]non-4-yl)(5-phénylfuran-2-yl)méthanone ;
la (1,4-diazabicyclo[3,2,2]non-4-yl)(5-(2-pyridyl)-furan-2-yl)méthanone ;
la (1,4-diazabicyclo[3,2,2]non-4-yl)(5-(3-pyridyl)-furan-2-yl)méthanone ;
la (1,4-diazabicyclo[3,2,2]non-4-yl)(5-(4-pyridyl)-furan-2-yl)méthanone ;
la (1,4-diazabicyclo[3,2,2]non-4-yl)(5-(furan-2-yl)-furan-2-yl)méthanone ;
la (1,4-diazabicyclo[3,2,2]non-4-yl)(5-(furan-3-yl)-furan-2-yl)méthanone ;
la (1,4-diazabicyclo[3,2,2]non-4-yl)(5-(thiophén-2-yl)furan-2-yl)méthanone ;
la (1,4-diazabicyclo[3,2,2]non-4-yl)(5-(thiophén-3-yl)furan-2-yl)méthanone ;
la (1,4-diazabicyclo[3,2,2]non-4-yl)(2-phényl-thiophén-4-yl)méthanone ;
la (1,4-diazabicyclo[3,2,2]non-4-yl)(2-(2-pyridyl)-thiophén-4-yl)méthanone ;
la (1,4-diazabicyclo[3,2,2]non-4-yl)(2-(3-pyridyl)-thiophén-4-yl)méthanone ;
la (1,4-diazabicyclo[3,2,2]non-4-yl)(2-(4-pyridyl)-thiophén-4-yl)méthanone ;
la (1,4-diazabicyclo[3,2,2]non-4-yl)(2-(furan-2-yl)-thiophén-4-yl)méthanone ;
la (1,4-diazabicyclo[3,2,2]non-4-yl)(2-(furan-3-yl)-thiophén-4-yl)méthanone ;
la (1,4-diazabicyclo[3,2,2]non-4-yl)(2-(thiophén-2-yl)thiophén-4-yl)méthanone ;
la (1,4-diazabicyclo[3,2,2]non-4-yl)(2-(thiophén-3-yl)thiophén-4-yl)méthanone ;
la (1,4-diazabicyclo[3,2,2]non-4-yl)(4-phénylfuran-2-yl)méthanone ;
la (1,4-diazabicyclo[3,2,2]non-4-yl)(4-(2-pyridyl)-furan-2-yl)méthanone ;
la (1,4-diazabicyclo[3,2,2]non-4-yl)(4-(3-pyridyl)-furan-2-yl)méthanone ;
la (1,4-diazabicyclo[3,2,2]non-4-yl)(4-(4-pyridyl)-furan-2-yl)méthanone ;
la (1,4-diazabicyclo[3,2,2]non-4-yl)(4-(furan-2-yl)-furan-2-yl)méthanone ;
la (1,4-diazabicyclo[3,2,2]non-4-yl)(4-(furan-3-yl)-furan-2-yl)méthanone ;
la (1,4-diazabicyclo[3,2,2]non-4-yl)(4-(thiophén-2-yl)furan-2-yl)méthanone ;
la (1,4-diazabicyclo[3,2,2]non-4-yl)(4-(thiophén-3-yl)furan-2-yl)méthanone ;
la (1,4-diazabicyclo[3,2,2]non-4-yl)(5-phényl-thiophén-2-yl)méthanone ;
la (1,4-diazabicyclo[3,2,2]non-4-yl)(5-(2-pyridyl)-thiophén-2-yl)méthanone ;
la (1,4-diazabicyclo[3,2,2]non-4-yl)(5-(3-pyridyl)-thiophén-2-yl)méthanone ;
la (1,4-diazabicyclo[3,2,2]non-4-yl)(5-(4-pyridyl)-thiophén-2-yl)méthanone ;

la (1,4-diazabicyclo[3,2,2]non-4-yl)(5-(furan-2-yl)-thiophén-2-yl)méthanone ;
la (1,4-diazabicyclo[3,2,2]non-4-yl)(5-(furan-3-yl)-thiophén-2-yl)méthanone ;
la (1,4-diazabicyclo[3,2,2]non-4-yl)(5-(thiophén-2-yl)thiophén-2-yl)méthanone ;
la (1,4-diazabicyclo[3,2,2]non-4-yl)(5-(thiophén-3-yl)thiophén-2-yl)méthanone ;
la (1,4-diazabicyclo[3,2,2]non-4-yl)(2-phénylfuran-4-yl)méthanone ;
la (1,4-diazabicyclo[3,2,2]non-4-yl)(2-(2-pyridyl)-furan-4-yl)méthanone ;
la (1,4-diazabicyclo[3,2,2]non-4-yl)(2-(3-pyridyl)-furan-4-yl)méthanone ;
la (1,4-diazabicyclo[3,2,2]non-4-yl)(2-(4-pyridyl)-furan-4-yl)méthanone ;
la (1,4-diazabicyclo[3,2,2]non-4-yl)(2-(furan-2-yl)-furan-4-yl)méthanone ;
la (1,4-diazabicyclo[3,2,2]non-4-yl)(2-(furan-3-yl)-furan-4-yl)méthanone ;
la (1,4-diazabicyclo[3,2,2]non-4-yl)(2-(thiophén-2-yl)furan-4-yl)méthanone ;
la (1,4-diazabicyclo[3,2,2]non-4-yl)(2-(thiophén-3-yl)furan-4-yl)méthanone ;
la (1,4-diazabicyclo[3,2,2]non-4-yl)(4-phényl-thiophén-2-yl)méthanone ;
la (1,4-diazabicyclo[3,2,2]non-4-yl)(4-(2-pyridyl)-thiophén-2-yl)méthanone ;
la (1,4-diazabicyclo[3,2,2]non-4-yl)(4-(3-pyridyl)-thiophén-2-yl)méthanone ;
la (1,4-diazabicyclo[3,2,2]non-4-yl)(4-(4-pyridyl)-thiophén-2-yl)méthanone ;
la (1,4-diazabicyclo[3,2,2]non-4-yl)(4-(furan-2-yl)-thiophén-2-yl)méthanone ;
la (1,4-diazabicyclo[3,2,2]non-4-yl)(4-(furan-3-yl)-thiophén-2-yl)méthanone ;
la (1,4-diazabicyclo[3,2,2]non-4-yl)(4-(thiophén-2-yl)thiophén-2-yl)méthanone, ou
la (1,4-diazabicyclo[3,2,2]non-4-yl)(4-(thiophén-3-yl)thiophén-2-yl)méthanone,

ou un diastéréoisomère, un énantiomère ou un sel pharmaceutiquement acceptable de celui-ci.

**16.** Composé selon l'une quelconque des revendications 1 à 15 destiné à être utilisé en thérapie.

**17.** Composé selon l'une quelconque des revendications 1 à 15 destiné à être utilisé comme médicament.

**18.** Utilisation d'un composé, tel que défini dans l'une quelconque des revendications 1 à 15, dans la fabrication d'un médicament destiné au traitement ou à la prophylaxie de troubles psychotiques, de troubles d'altération intellectuelle, de maladies ou d'affections humaines dans lesquelles l'activation du récepteur nicotinique $\alpha$7 est bénéfique, de la maladie d'Alzheimer, du déficit d'apprentissage, du déficit cognitif, du déficit attentionnel, de la perte de mémoire, de la démence à corps de Lewy, du trouble de l'attention et de l'hyperactivité, de l'anxiété, de la schizophrénie, de la manie ou de la psychose maniaco-dépressive, de la maladie de Parkinson, de la maladie de Huntington, du syndrome de Tourette, des troubles neurodégénératifs dans lesquels il y a une perte de la synapse cholinergique, du syndrome du décalage horaire, du sevrage tabagique, du tabagisme, y compris celui qui résulte de l'exposition à des produits contenant de la nicotine, de la douleur, de la colite ulcéreuse ou du syndrome de l'intestin irritable.

**19.** Composition pharmaceutique comprenant un composé de formule I, tel que défini dans l'une quelconque des revendications 1 à 15, conjointement à au moins un excipient ou un diluant pharmaceutiquement acceptable.

**20.** Procédé de préparation d'un composé de formule I, tel que défini dans l'une quelconque des revendications 1 à 15, **caractérisé en ce qu'**il comprend :

la réaction d'un composé de formule VI :

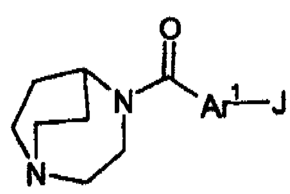

VI

dans laquelle J représente halogène ou un substituant $OSO_2CF_3$ dans la position du cycle $Ar^1$ dans laquelle la liaison avec le cycle $Ar^2$ est formée avec un composé organométallique de formule VII:

**$Ar^2$-M** **VII**

en présence d'un catalyseur organométallique et d'un solvant.

**21.** Composé de formule VI:

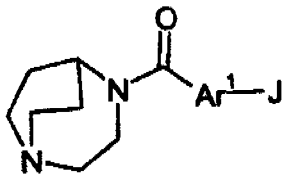

VI

dans laquelle :

$Ar^1$ est un cycle benzène, furane ou thiophène ;
J est halogène ou $OSO_2CF_3$, à condition que, lorsque $Ar^1$ est un cycle benzène, J ne puisse représenter qu'halogène ou $OSO_2CF_3$ en position méta ou para du groupement carboxamide ; ou un énantiomère de celui-ci ou des sels pharmaceutiquement acceptables de celui-ci.

**22.** Composé selon la revendication 21, choisi parmi :

la (1,4-diazabicyclo[3,2,2]non-4-yl)(5-bromofuran-2-yl)méthanone ;
la (1,4-diazabicyclo[3,2,2]non-4-yl)(5-bromothio-phén-2-yl)méthanone ;
la (1,4-diazabicyclo[3,2,2]non-4-yl)(3-bromophényl)-méthanone ;
la (1,4-diazabicyclo[3,2,2]non-4-yl)(4-bromophényl)-méthanone ;
la (1,4-diazabicyclo[3,2,2]non-4-yl)(3-iodophényl)-méthanone ;
la (1,4-diazabicyclo[3,2,2]non-4-yl)(4-iodophényl)-méthanone ;
la (1,4-diazabicyclo[3,2,2]non-4-yl)(4-bromothio-phén-2-yl)méthanone ; et
la (1,4-diazabicyclo[3,2,2]non-4-yl)(5-bromothio-phén-3-yl)méthanone ;

ou un énantiomère de celui-ci, ou un sel pharmaceutiquement acceptable de celui-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0058311 A
- FR 2809731
- FR 2809732
- US 4895543 A
- EP 215650 A


### Non-patent literature cited in the description


- Nicotinic Acetylcholine Receptors: Molecular Biology, Chemistry and Pharmacology. **MCDONALD et al.** Annual Reports in Medicinal Chemistry. Academic Press Inc, 1995, vol. 30, 41-50
- **WILLIAMS et al.** Neuronal Nicotinic Acetylcholine Receptors. *Drug News & Perspectives,* 1994, vol. 7, 205-223
- *Organic Syntheses,* 1963, vol. 4, 68
- *J. Org. Chem.,* 1995, vol. 60, 7508
- *J. Gen. Chem. USSR,* 1964, 2222-2228
- Protecting groups in Organic Synthesis. Greene and Wuts, 1999
- **MARTINO-BARROWS ; KELLAR.** *Mol Pharm,* 1987, vol. 31, 169-174
- **DELEAN A ; MUNSON P J ; RODBARD D.** *Am. J. Physiol.,* 1977, vol. 235, E97-E102